# EUROPEAN PATENT APPLICATION

(11) **EP 2 105 142 A1**
(43) Date of publication of application: **30.09.2009**
(21) Application number: 08153443.0
(22) Date of filing: 27.03.2008
(51) Int. Cl.: A61K 38/17

(54) **Means and methods for influencing electrical activity of cells**

(71) Applicant: Academisch Medisch Centrum bij de Universiteit van Amsterdam, 1105 AZ Amsterdam (NL)
(72) Inventor: The designation of the inventor has not yet been filed
(74) Representative: Hatzmann, Martin

(57) **Abstract**

The invention provides means and methods for providing a cell with a spontaneous electrical activity and means and methods for increasing the depolarization rate of a cell having a spontaneous electrical activity. Means and methods are provided comprising:
- providing a cell with a compound capable of providing and/or increasing a pacemaker current If, and
- diminishing electrical coupling between said cell and surrounding cells and/or reducing the inward rectifier current I_{K1} of said cell.

## Description

The invention relates to the fields of biology and medicine.

Various kinds of animal cells exhibit electrical activity. For instance, information is transferred by cells of the nervous system via electrical signals. Gastrointestinal motility involves electrical activity of gastrointestinal cells, whereas glucose-induced release of insulin involves electrical activity of pancreatic islet cells. Another important biological function involved with electrical signals is the heartbeat. The heartbeat is driven by action potentials (APs) generated spontaneously in the sinoatrial (SA) node. Old age and a variety of cardiovascular disorders may disrupt normal SA node function. This can result in disease-causing slow heart rates in conjunction with fast heart rates, called "sick sinus syndrome". Due to aging of the general population and an associated rise in the prevalence of cardiovascular disease, the prevalence and clinical impact of this syndrome are likely to increase. Currently, cardiac rhythm disorders such as sick sinus syndrome (SSS) and AV nodal block (AVB) are usually treated by electronic pacemakers. Electronic pacemakers are of great value in the therapy of cardiac conduction disease. These devices have become more and more sophisticated over the past years, but there are shortcomings. Items that need improvement include the lack of autonomic modulation of the heart rate, the limited battery life, unstable electrode position, and electronic or magnetic interference. Creating an autonomically controlled biological pacemaker would solve these limitations. As used herein, the term "biological pacemaker" is also referred to as "biopacemaker".
Currently, bio-engineered pacemakers are experimentally combined with electronic pacemakers. Advantages of such combinations over electronic pacemakers comprise improved autonomic modulation and extended battery lives of the combined entity. For instance, patent application WO 2007/014134 describes a pacemaker system comprising an electronic pacemaker and a biological pacemaker, wherein the biological pacemaker comprises cells that functionally express a chimeric hyperpolarization-activated, cyclic nucleotide-gated (HCN) ion channel. However, this chimeric HCN channel exhibited bursts of tachyarrhythmias both *in vitro* and *in vivo* (Plotnikov AN et al. Heart Rhythm 2008). Proof of concept was obtained by implanting the tandem biological and electronic pacemaker combination in dogs (wild-type HCN2 and an engineered HCN2 mutant; the mutant demonstrated improved channel kinetics however with a lower level of gene expression). Pacemaker activity was measured, whereby both components complemented each other. When the biological component slowed, the electronic component took over. Subsequently, when the biological component slowed down in rate, the electronic component started to fire (while the electronic pacemaker did not fire if the rate of the biological component was fast enough). Hence, the electronic pacemaker component was needed in order to provide sufficient pacemaker function.

A drawback of this pacemaker combination is the fact that two separate entities have to be brought into a heart. Moreover, disadvantages involving limited battery life (although battery life was extended as compared to the use of an electronic pacemaker alone), unstable electrode position and electronic or magnetic interference are still present.

It would be advantageous to use a biological pacemaker only, since the above mentioned disadvantages of the electronic pacemaker component would be overcome and autonomic modulation would be possible. However, until now biological pacemakers do not provide sufficient heart function. Slow beating rates and periods of complete cessation of beating are observed. Furthermore, biological biopacemaker rhythms exhibited unexplained large variations in beating rates (cycle lengths) [Cai et al (2007); Bucchi et al (2006)].

It is an object of the present invention to provide means and methods for providing cells with spontaneous electrical activity and/or for increasing spontaneous electrical activity of cells having electrical activity, so that biological functions involving spontaneous electrical activity are provided, increased and/or restored. It is a further object of the present invention to provide improved biological pacemakers.

Accordingly, the present invention provides a method for providing a cell with a spontaneous electrical activity and/or increasing the depolarization rate of a cell having a spontaneous electrical activity, the method comprising:
- providing a cell with a compound capable of providing and/or increasing a pacemaker current I_{f}, and
- diminishing electrical coupling between said cell and surrounding cells and/or reducing the inward rectifier current I_{K1} of said cell.

Spontaneous electrical activity of a cell is herein defined as a firing capability, involving a spontaneous (i.e., without the need of an external electrical trigger) alteration of a cell's membrane potential in time (hyperpolarization/depolarization), resulting in transmission of excitation between cells (firing).
As used herein, a cell having spontaneous electrical activity is called a pacemaker cell.

With a method according to the present invention, a cell is provided with a spontaneous electrical activity and/or the spontaneous electrical activity of a cell is enhanced. This way, biological functions involving spontaneous electrical activity of cells are obtained, improved and/or restored.
One important biological function that is improved with a method according to the present invention is heartbeat. Without limiting the scope of the invention, cardiac applications are discussed in more detail.

In the heart, the pacemaker current I_{f} is naturally found in cells of the SA node. The SA node is a heterogonous structure composed of specialized cardiomyocytes and a high level of connective tissue. The activity in this node is driven by a spontaneous change in the membrane potential, called the slow diastolic depolarization or phase 4 depolarization. This phase 4 depolarization results in the formation of action potentials, thereby triggering the contraction of the heart. A major current underlying this process is the "funny current" or I_{f}. A family of hyperpolarization-activated cyclic nucleotidegated (HCN) channels underlies this inward current. There are four HCN isoforms (HCN1, HCN2, HCN3 and HCN4) which are all expressed in the human heart, but expression levels vary among regions. The activity of HCN channels is controlled by the cyclic adenosine monophosphate (cAMP)-binding site which allows alteration of activation kinetics by beta-adrenergic and muscarinic stimulation. By this mechanism, channel activity is increased or decreased. This plays an important role in the autonomic regulation of heart rate.
In a method according to the invention, a cell is provided with a compound capable of providing and/or increasing a pacemaker current I_{f}. In one preferred embodiment said compound comprises a hyperpolarization-activated cyclic nucleotide-gated (HCN) channel or a functional equivalent thereof. HCN channels underlie the Iₕ current (termed also I_{f} in the heart and Iq in the brain). The most prominent function proposed for this current is the generation of spontaneous rhythmic activity in heart, brain and insulin-secreting cells. Therefore, If has been called 'pacemaker current' and HCN channels have been designated 'pacemaker channels'. Increasing HCN current results in increased diastolic depolarization, thereby enhancing the basal firing frequency.
A HCN channel is a sodium/potassium cation channel that is activated by membrane hyperpolarization. Activation of HCN channels results in an inward current carried by sodium/potassium which causes depolarization of the membrane potential. Hence, administration of HCN to a cell provides said cell with a pacemaker current or increases the pacemaker current of said cell.
The isoforms of HCN are capable of forming heterotetrameric complexes. Moreover, it is possible to design a HCN channel which comprises components of at least two different HCN forms. Alternatively, or additionally, one or more components of a HCN channel is/are modified, added or deleted in order to obtain a HCN-derived cation channel. As used herein, the term HCN or functional equivalent thereof embraces such embodiments. A functional equivalent of a HCN channel is defined as a compound which has at least one same property as HCN in kind, not necessarily in amount. A functional equivalent of a HCN channel is capable of being activated by membrane hyperpolarization; this activation results in an inward current carried by sodium/potassium which causes depolarization of a membrane potential. A functional equivalent of a HCN channel is for instance formed by building a cation channel using elements of different HCN isoforms. For instance, HCN1 exhibits rapid activation kinetics, whereas HCN4 exhibits a stronger cAMP response. In one embodiment HCN1 and HCN4 elements are therefore combined in order to obtain a HCN channel with an improved combination of activation kinetics/ cAMP response properties.
Alternatively, or additionally, a functional equivalent of HCN comprises at least one modified HCN sequence, as compared to natural HCN. In one embodiment a functional equivalent of HCN is provided through amino acid deletion and/or substitution, whereby an amino acid residue is substituted by another residue, such that the overall functioning is not seriously affected. Preferably, however, a HCN channel is modified such that at least one property of the resulting compound is improved as compared to wild type HCN. In a preferred embodiment a HCN mutant is used which is designed to shift the I_{f} activation curve to depolarized potentials. Such shift results in I_{f} current being more easily activated, i.e., at a potential which lies more closely to the resting membrane potential. Said shift is preferably essentially similar to the shift upon cAMP stimulation which normally results from beta-adrenergic stimulation. In one embodiment, a cell is provided with a functional equivalent of HCN as well as with wild-type HCN. These strategies further increase inward currents and result in faster beating.

In one particularly preferred embodiment a compound capable of providing and/or increasing a pacemaker current comprises a nucleic acid sequence encoding at least one HCN channel or a functional equivalent thereof. As used herein, the term "nucleic acid" encompasses natural nucleic acid molecules, such as for instance DNA, RNA and mRNA, as well as artificial sequences such as for instance a DNA/RNA helix, peptide nucleic acid (PNA), locked nucleic acid (LNA), et cetera. Many methods are known in the art for providing a cell with a nucleic acid sequence. For instance, calcium phosphate transfection, DEAE-Dextran, electroporation or liposome-mediated transfection is used. Alternatively, direct injection of the nucleic acid is employed. Preferably however said nucleic acid is introduced into the cell by a vector, preferably a viral vector. Most preferably long-term expression vectors are used, such as for instance Adeno Associated Vectors (AAV) or retroviral vectors, such as a lentiviral vector. Although adenoviral vectors (Ad) efficiently transduce cells, their usefulness as a therapeutic tool is limited, because they mediate only transient gene expression. An advantage of lentiviral vectors is that they integrate into the host genome. This induces long-term transgene expression, and renders these vectors ideal candidates to manage a chronic condition, such as sick sinus syndrome. Lentiviral vectors, for instance derived from human immunodeficiency virus (HIV) are therefore preferred. AAV vectors have the advantage that they are better suitable to scale up production capacity for therapeutic applications, for instance. Cardiac specific AAV serotypes such as AV-1, AAV-6, AAV-8 and AAV-9 are therefore also preferred.

Various terms are known in the art which refer to introduction of nucleic acid into a cell by a vector. Examples of such terms are "transduction", "transfection" or "transformation". Techniques for generating a vector with a nucleic acid sequence of interest and for introducing said vector into a cell are known in the art. If desired, it is possible to use marker genes in order to determine whether a nucleic acid of interest has been introduced into a cell, as is well known in the art. See for instance the well known handbook of Sambrook and Russell (Molecular cloning, a laboratory manual, third edition, 2001 Cold Spring Harbour Laboratory Press, Cold Spring Harbour, New York).

One preferred embodiment thus provides a method according to the invention, wherein a cell is provided with a hyperpolarization-activated cyclic nucleotide-gated (HCN) channel or a functional equivalent thereof, or a nucleic acid sequence coding therefore. In one embodiment said nucleic acid sequence encodes a wild type HCN. In one preferred embodiment, however, said nucleic acid sequence encodes a HCN mutant which is designed to shift the I_{f} activation curve to depolarized potentials. As explained above, such shift results in I_{f} current being more easily activated, i.e., at a potential which lies more closely to the resting membrane potential. In another preferred embodiment, one or more nucleic acid sequence(s) encoding a HCN mutant and wild type HCN is/are used.

Another preferred embodiment provides a method according to the present invention, wherein the basal cAMP level within said cell is enhanced. An increase in intracellular cAMP levels directly shifts I_{f} activation towards more depolarized potentials and it also stimulates intracellular Ca²⁺ handling via PKA dependent phosphorylation of involved proteins, such as the L-type Calcium channel, SERCA end RyR. This increases beating rates. In one embodiment the basal cAMP level of a cell is enhanced by increasing the amount and/or activity of a cAMP producing enzyme within said cell. In this embodiment an increased amount of cAMP is produced by said enzyme, resulting in increased pacemaker activity. Said enzyme preferably comprises an adenylate cyclase (AC), more preferably adenylate cyclase-1 and/or adenylate cyclase-8. These enzymes are Ca/calmodulin stimulated ACs and provide a crucial link between I_{f} based impulse formation and spontaneous Ca²⁺ oscillations, a mechanism that also importantly contributes to normal SA node impulse formation. An increased amount and/or activity of AC therefore improves biopacemaker function.
The amount of a cAMP producing enzyme in a cell is increased using any method known in the art. For instance, a nucleic acid sequence encoding a cAMP producing enzyme, or a functional equivalent thereof which is also capable of increasing cellular cAMP levels, is introduced into said cell, for instance using a (viral) vector. Said nucleic acid sequence is preferably operably linked to a promoter. If desired, an inducible promoter is chosen, so that the amount of expression can be regulated at will. This is, however, not necessary. Of course, alternative methods known in the art for increasing an amount of an enzyme of interest are suitable as well. The choice for a certain method depends on the specific circumstances.
Various methods for increasing the activity of a cAMP producing enzyme are also known in the art. For instance an activator, or a nucleic acid sequence encoding an activator, is administered to a cell comprising said cAMP producing enzyme, resulting in an enhanced activity of said enzyme.

In another embodiment, the basal cAMP level within a cell is enhanced by reducing the amount and/or activity of an enzyme involved with cAMP breakdown. Said enzyme preferably comprises a phosphodiesterase (PDE). Tissue-specific enzyme subtypes of PDE appear to be involved in subcellular regulation of cAMP mediated by cAMP breakdown. In particular, PDE4 is involved in beta-adrenergic signaling of both sarcolemmal ion channels (e.g., HCN) and sarcolplasmatic reticulum (SR) Ca²⁺ handling proteins (e.g., SERCA). Suppression of PDE activity therefore improves biopacemaker function.
Various methods are known in the art for reducing the amount and/or activity of a given enzyme. For instance, an antisense nucleic acid sequence and/or siRNA is administered to a cell in order to suppress expression of said enzyme. It is also possible to add an enzyme inhibitor, or a nucleic acid sequence coding therefore. Increasing the amount and/or activity of such enzyme inhibitor thus indirectly decreases the amount and/or activity of said enzyme. A non-limiting example of a PDE inhibitor is PI3K_{Y}, which is an upstream modulator of PDE activity. Increasing the amount and/or activity of PI3K_{Y} results in a reduced amount and/or activity of PDE.
In one preferred embodiment a cell is provided with:
- an siRNA and/or an antisense nucleotide sequence against a phosphodiesterase; and/or
- a nucleic acid sequence or a functional equivalent thereof encoding a phosphodiesterase with a diminished function as compared to wild type phosphodiesterase. Such phosphodiesterase with a diminished function is less capable of inducing cAMP breakdown. It has preferably retained its capability of binding free cAMP, so that free cAMP is bound but not, or to a lesser extent, degraded. A cell is preferably provided with a nucleic acid sequence or a functional equivalent thereof encoding a phosphodiesterase with a diminished function as compared to wild type phosphodiesterase, wherein said nucleic acid sequence or functional equivalent thereof encodes a phosphodiesterase with a dominant diminished function as compared to wild type phosphodiesterase. Such phosphodiesterase is preferably capable of suppressing the activity of wild type phosphodiesterase, for instance by forming a complex with a wild type phosphodiesterase thereby at least in part inhibiting its activity.

I_{f} is not the only current contributing to the pacemaker cell membrane potential. Other inward and outward currents are involved as well. Any increase in inward and/or decrease in outward current initiates or accelerates the process of phase 4 depolarization. Inward and outward currents of a pacemaker cell are often promoted or blocked by electrical interactions with the surrounding tissue. Interfering with electrical coupling between a pacemaker cell and surrounding cells therefore significantly influences these inward and outward currents, and pacemaker activity.

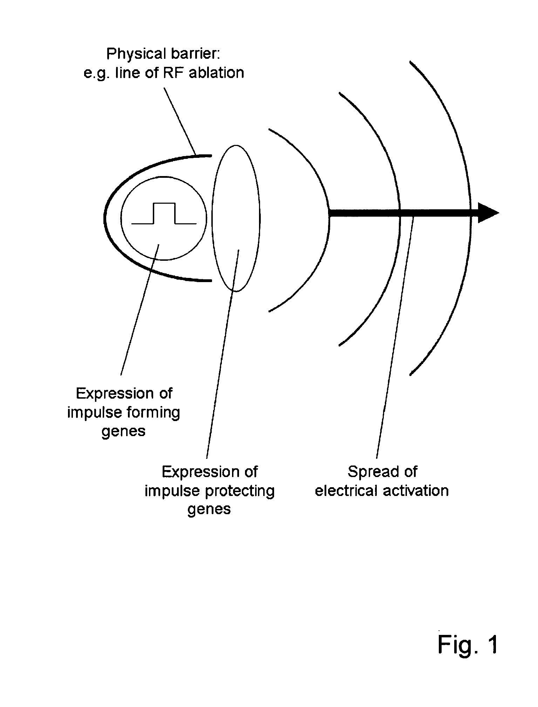

In one preferred embodiment of the present invention a cell is provided with a compound capable of providing and/or increasing a pacemaker current I_{f}, and electrical coupling between said cell and surrounding cells is diminished. According to the present invention, partial electrical uncoupling, reducing the electrical load and/or physical uncoupling stabilizes the function of a pacemaker cell. As a consequence the required size of a pacemaker region is reduced. Paradoxically, the spread of electrical activation is improved by partial uncoupling. Without being bound to theory, an explanation for these findings lies within the effects of the inward rectifier current (I_{K1}) in the surrounding tissue, which acts to maintain the resting membrane potential, thereby counteracting depolarization of the pacemaker region. Partial uncoupling alleviates this load-mismatch, because it isolates the small region of pacemaker cells from these effects of I_{K1} from the large surrounding region. This reduces the amount of pacemaker current which is required for successful generation of spontaneous action potentials in the pacemaker region and subsequent spread of activation to the surrounding regions.
One embodiment of the present invention provides a method for providing a cell with a spontaneous electrical activity and/or increasing the depolarization rate of a cell having spontaneous electrical activity, wherein the electrical coupling between said cell and surrounding cells is diminished by providing a barrier between said cell and surrounding cells. Said barrier preferably comprises a cell with a reduced conductor capacity as compared to the same kind of cell in a natural situation, so that the electrical coupling between a pacemaker cell and the surrounding region is diminished. In one embodiment, said barrier comprises fibrotic cells. As used herein, a fibrotic cell is defined as an injured cell that has a lower capability to conduct or generate an electrical impulse as compared to normal, healthy cells. Preferably, said fibrotic cell has lost its capability to conduct or generate an electrical impulse. Fibrotic cells are obtained in various ways. Preferably, surrounding cells are rendered fibrotic by heating them with a heating element with a temperature of at least 55°C, preferably at least 60°C or cooling them with a cooling element with a temperature of at most -75°C, preferably at most -80°C. One embodiment therefore provides a method according to the invention for providing a cell with a spontaneous electrical activity and/or increasing the depolarization rate of a cell having spontaneous electrical activity, wherein the electrical coupling between said cell and surrounding cells is diminished by heating surrounding cells with a device having a temperature of at least 55°C. Said cells are preferably heated with a device having a temperature of about 60°C. Preferably, said surrounding cells are heated with a device having a temperature of between 50°C and 75°C, more preferably between 50°C and 65°C. In one particularly preferred embodiment said surrounding cells are heated with a device having a temperature of between 55°C and 60°C. In one preferred embodiment (catheter-based) radiofrequency ablation is used. With this technique, a targeted area is gently warmed to about 60°C to completely disrupt cell-to-cell electrical connections. A non-limiting example of impulse protection based on physical uncoupling in combination with partial uncoupling or load reduction is schematically depicted in Figure 1.
Further provided is therefore a method according to the invention, wherein said barrier comprises cells which have been heated with a device having a temperature of at least 50°C, preferably with a device having a temperature of between 50 and 65°C, more preferably with a device having a temperature of between 55 and 60°C. Yet another embodiment provides a method according to the invention for providing a cell with a spontaneous electrical activity and/or increasing the depolarization rate of a cell having spontaneous electrical activity, wherein the electrical coupling between said cell and surrounding cells is diminished by cooling surrounding cells, preferably to about -80°C. In one preferred embodiment cryo ablation is used. With this technique, a targeted area is gently cooled, preferably to about -80°C, to disrupt cell-to-cell electrical connections.

In one particularly preferred embodiment the electrical coupling between a pacemaker cell and surrounding cells is diminished such that the electrical impulse of a firing pacemaker cell will not, or to a significantly lesser extent, be conducted into a certain direction. Hence, preferably, electrical connections between a pacemaker cell and surrounding tissue are primarily present in one or several directions, whereas electrical impulses to at least one other direction are preferably diminished. This is for instance performed by providing a conductor barrier which partly surrounds said pacemaker cell. Said barrier preferably has a shape that allows for diminishing, preferably blocking, electrical connections between said cell and the surrounding tissues in at least one direction. In a particularly preferred embodiment, electrical connections to the surrounding tissues are blocked in at least one direction with respect to the plane that runs parallel to the heart surface. A non-limiting example is schematically depicted in Figure 1: an electrical impulse of firing pacemaker cells is conducted to a certain direction because other directions are blocked by a barrier (for instance fibrotic cells).

Another way of diminishing electrical coupling between a pacemaker cell and surrounding cells is reduction of the amount and/or activity of gap junction proteins connecting said cell and surrounding cells. A gap junction is a junction between cells that allows different molecules and ions, mostly small intracellular and intercellular signaling molecules (intracellular and intercellular mediators), to pass freely between cells. A gap junction comprises protein channels in cell membranes that allow ions and small molecules to pass between adjacent cells. The protein channels that make up gap junctions usually consist of two connexons. One connexon resides in the membrane of one cell. It aligns and joins the connexon of the neighboring cell, forming a continuous aqueous pathway by which ions and small molecules can freely pass (passively) from one cell to the other. Connexons usually consist of six subunits called connexins. The connexin genes have been highly conserved during evolution. In some cells the connexons are formed of six identical connexins or of some combination of two different connexins.

Reducing the amount and/or activity of gap junction proteins connecting a pacemaker cell and surrounding cells diminishes electrical coupling between said cells. Further provided is therefore a method according to the invention, wherein the electrical coupling between said cell and surrounding cells is diminished by reducing the amount and/or activity of gap junction proteins connecting said cell and surrounding cells. In ventricular myocardial tissue, the gap junction protein connexin 43 is highly expressed and in atrial myocardium both connexin 40 and 43 are abundantly present. In order to diminish electrical coupling between cardiac cells, a cardiac pacemaker cell is therefore preferably provided with a gap junction protein with a diminished conductor capacity as compared to connexin 43 and/or connexin 40. Since several connexins assemble together in order to form a connexon, gap junction proteins with a diminished conductor capacity will assemble with wild type connexins in a cell so that a connexon is formed which has a lower conductor capacity.
Further provided is therefore a method according to the invention, wherein the electrical coupling between a pacemaker cell and surrounding cells is diminished by providing said cell with a gap junction protein with a diminished conductor capacity as compared to connexin 43 and/or connexin 40.
In one embodiment the amount and/or activity of connexin 43 and/or connexin 40 of a pacemaker cell is reduced. A method according to the invention, wherein the electrical coupling between a pacemaker cell and surrounding cells is diminished by reducing the amount and/or activity of connexin 43 and/or connexin 40 of said cell is therefore also provided. The amount of connexin 43 and/or connexin 40 in a cell is for instance reduced using antisense nucleic acid and/or siRNA which is capable of reducing expression of connexins. The activity of connexin 43 and/or connexin 40 is for instance reduced by administration of connexins with a lower conductor capacity, or nucleic acid coding therefore, as described before.
One particularly preferred embodiment provides a method for providing a cell with a spontaneous electrical activity and/or increasing the depolarization rate of a cell having a spontaneous electrical activity, comprising providing said cell with a compound capable of providing and/or increasing a pacemaker current I_{f}, and providing said cell with:
- an siRNA and/or an antisense nucleotide sequence against connexin 43; and/or
- an siRNA and/or an antisense nucleotide sequence against connexin 40; and/or
- a nucleic acid sequence or a functional equivalent thereof encoding a connexin with a lower conductor capacity than the conductor capacity of connexin 43; and/or
- a nucleic acid sequence or a functional equivalent thereof encoding a connexin with a lower conductor capacity than the conductor capacity of connexin 40.
   As used herein, a connexin with a lower conductor capacity than the conductor capacity of connexin 40 or connexin 43 is defined as a connexin or a functional equivalent thereof which is capable of assembling with other connexins in order to form a connexon through which ions and other small molecules can pass from one cell to the other, wherein less molecules are capable of passing through the resulting connexon within a given time frame as compared to a connexon which is solely composed of wild type connexins 40 and/or 43.
   Non-limiting, preferred examples of connexins with a lower conductor capacity than the conductor capacity of connexin 40 or connexin 43 are connexin 30.2, connexin 45 and connexin43Δ, a mutated connexin 43 with dominant negative characteristics, for example as described by (Krutovskikh VA Molecular carcinogenesis 1998*).* Connexin 30.2 is an SA node-specific connexin. Further provided is therefore a method according to the invention, wherein the electrical coupling between a pacemaker cell and surrounding cells is diminished by providing said cell with connexin 30.2 and/or connexin 45 and/or connexin43Δ and/or a functional equivalent thereof.
   In yet another embodiment a cell is provided with a spontaneous electrical activity and/or the depolarization rate of a cell having a spontaneous electrical activity is increased by providing said cell with a transcription factor capable of reducing connexin 43 expression and/or connexin 40 expression so that fewer connexons are formed. Said transcription factor preferably comprises TBX3.
   Spontaneous electrical activity is also provided or enhanced by administration of a beta-subunit for a voltage gated potassium channel (e.g. MirP1) to a cell. Such beta-subunit is capable of forming a complex with HCN, thereby increasing the pacemaker current I_{f}. Preferably, a cell is provided with a nucleic acid sequence encoding said beta-subunit. Further provided is therefore a method according to the invention, further comprising providing a cell with a nucleic acid sequence or a functional equivalent thereof encoding a beta-subunit for a voltage gated potassium channel.
   In one aspect of the invention, protection of impulse formation is achieved by a reduction in the electrical load imposed by cells that surround a pacemaker cell. Said load is preferably reduced by shifting the resting membrane potential of said surrounding cells to more positive potential. This will subsequently result in a shift to more positive potentials of the resting membrane potential (called the maximal diastolic potential, MDP) of a pacemaker cell. This enhances basal pacing rates, as the MDP is closer to the threshold potential at which the pacemaker AP is initiated, thereby stabilizing basal pacemaker firing rates. A direct load reduction is preferably achieved by reducing the repolarizing inward rectifier potassium current I_{K1}.
   In one preferred embodiment of the present invention, the inward rectifier current I_{K1} of a pacemaker cell is preferably reduced by providing said pacemaker cell with
- an siRNA and/or an antisense nucleotide sequence against an inwardly-rectifying channel; and/or
- a nucleic acid sequence or a functional equivalent thereof encoding an inwardly-rectifying channel with a diminished function as compared to the same kind of inwardly-rectifying channel in a wild type form.
   An siRNA and/or an antisense nucleotide sequence against an inwardly-rectifying channel is an siRNA and/or an antisense nucleotide sequence comprising a sequence which is complementary to a nucleic acid sequence encoding at least one protein of said inwardly-rectifying channel. Non-limiting examples of inwardly-rectifying channel are Kir 2.1, Kir2.2 and Kir3.1. When a cell has been provided with an siRNA and/or an antisense nucleotide sequence against such inwardly-rectifying channel, less proteins of said inwardly-rectifying channel will be expressed, resulting in a lower amount of inwardly-rectifying channels in said cell. This way, the inward rectifier current I_{K1} is reduced. In one particularly preferred embodiment said inwardly-rectifying channel comprises a Kir2.1 channel. Reducing the amount and/or activity of a Kir2.1 channel significantly reduces the inward rectifier current I_{K1}.

A method according to the invention is, amongst other things, particularly suitable for inducing and/or increasing spontaneous electrical activity in cardiac cells. This way, a biological pacemaker is provided, enabling stable long-term function at a physiological heart rate and enabling autonomic modulation, thereby circumventing regulation difficulties of electronic pacemakers. Further provided is therefore a method according to the invention for providing a cell with a spontaneous electrical activity and/or increasing the depolarization rate of a cell having a spontaneous electrical activity, wherein said cell is present in, or brought into, atrial or ventricular myocardium.
In one preferred embodiment said cell is present in, or brought into, an atrium of a heart. An electrical impulse of a cell that fires in this area of the heart is conducted via the atrioventricular node (AV node) to the ventricles of the heart. The AV node is capable of conducting a limited amount of electrical impulses. Hence, if the firing activity of cells in an atrium is too high and/or uncontrolled, the other parts of the heart are protected against an overload of electrical impulses, thereby avoiding heart rhythm disorders resulting in ventricular fibrillation or other lethal cardiac arrhythmias. This embodiment therefore provides an extra safety measure. Of course, this embodiment is only preferred if the AV node of a subject's heart functions properly. In one embodiment, an atrial biological pacemaker is generated by providing at least one atrial cell with a spontaneous electrical activity with a method according to the invention, and/or by increasing the depolarization rate of at least one atrial cell with a method according to the invention. Such atrial biological pacemaker is particularly suitable for treating sick sinus syndrome. Hence, the invention provides a method wherein a cardiovascular disorder, preferably sick sinus syndrome, is treated with an atrial biological pacemaker according to the invention. In one embodiment, an atrial biological pacemaker according to the invention is used without the use of an electronic pacemaker. This embodiment is particularly suitable for treating sick sinus syndrome.
If desired, however, an atrial biological pacemaker according to the invention is combined with an electronic pacemaker. Such combination is for instance suitable for treating AV nodal block, because in this case the electrical impulse of firing atrial cells will have difficulties in reaching the ventricles. A combination of a biological pacemaker according to the invention and an electronic pacemaker has improved properties as compared to the current experimental combinations of a biological pacemaker and an electronic pacemaker, amongst other things because the properties of a biological pacemaker according to the invention are improved as compared to conventional biological pacemakers. For instance, (autonomic modulation of) the heart rate is improved. Hence, one embodiment of the invention provides a method wherein a cardiovascular disorder, preferably AV nodal block, is treated with a combination of an atrial biological pacemaker according to the invention and an electronic pacemaker.

It is, of course, also possible to generate a ventricular biological pacemaker with a method according to the present invention. A ventricular biological pacemaker is generated by providing at least one ventricular cell (i.e., a cell located in the ventricular compartments, such as ventricular working myocardial cells or cells of the specialized conduction system) with a spontaneous electrical activity with a method according to the invention, and/or by increasing the depolarization rate of at least one ventricular cell with a method according to the invention. A ventricular biological pacemaker is for instance preferred when the AV node of a subject's heart does not function properly (or is at risk of not functioning properly). Hence, if a subject suffers from AV nodal block, a ventricular biological pacemaker according to the invention is preferred. The invention therefore provides a method wherein a cardiovascular disorder, preferably AV nodal block, is treated with a ventricular biological pacemaker according to the invention.
Also when the AV node of a subject's heart functions properly, the use of a ventricular biological pacemaker according to the present invention is advantageous because it provides an additional safety measure, since possible diminished function of the AV node in the future will not affect the biological pacemaker function.
In one embodiment, a ventricular biological pacemaker according to the invention is used without the use of an electronic pacemaker. If desired, however, a ventricular biological pacemaker according to the invention is combined with an electronic pacemaker. As described above, a combination of a biological pacemaker according to the invention and an electronic pacemaker has improved properties as compared to the current experimental combinations of a biological pacemaker and an electronic pacemaker, amongst other things because the properties of a biological pacemaker according to the invention are improved as compared to conventional biological pacemakers. For instance, (autonomic modulation of) the heart rate is improved. Hence, one embodiment of the invention provides a method wherein a cardiovascular disorder is treated with a combination of a ventricular biological pacemaker according to the invention and an electronic pacemaker.

A method according to the invention is suitable for providing or increasing spontaneous electrical activity in a cell of interest. In one embodiment gene therapy is applied wherein a cell of a subject, such as for instance a cardiac cell, is modified. This is for instance performed by providing a cell of a subject, preferably a cardiac cell, with a vector according to the invention, preferably a (lenti)viral vector, which vector comprises at least one nucleic acid sequence for providing said cell with spontaneous electrical activity and/or for improving the spontaneous electrical activity of said cell. In another embodiment, however, a dysfunctional organ or tissue, such as for instance a subject's heart, is provided with a cell wherein spontaneous electrical activity has been provided or enhanced with a method according to the invention. Preferably, said cell comprises a stem cell or progenitor cell.
Stem cells provide an alternative delivery platform or pacemaker source, especially when upregulation or downregulation of multiple genes is desired to improve overall pacemaker function. It is possible to use undifferentiated stem cells as well as differentiated stem cells. For cardiovascular applications in human individuals, human mesenchymal stem cells (hMSCs, undifferentiated) and/or human cardiac myocyte progenitor cells (hCMPCs, either differentiated or undifferentiated) are preferably used. As used herein, the term "stem cell" also encompasses progenitor cells. *Ex vivo* gene transfer provides an efficient strategy to introduce at least one nucleic acid sequence which allows for the creation of a homogeneous stem cell population with optimal pacemaker characteristics. If needed, multiple genes are easily introduced into stem cells. The risk of immunogenic rejection is maximally reduced by the use of autologous cells. Preferably, *ex vivo* lentiviral gene transfer is used because lentiviral vectors efficiently transduce cells and integrate into the host genome, allowing stable, long-term transgene expression.
Undifferentiated stem cells are easier to culture and to expand, and they are also immunoprivileged. However, these cells only function as a delivery system (for instance of an inward pacemaker current, as described hereinbefore). These cells lack the complete set of ion channels involved in membrane hyperpolarization and generation of cardiac action potentials (APs). Connexin proteins, importantly involved in electrical coupling and cell-to-cell transmission of electrical impulse, are therefore preferably present in both achieving the required membrane hyperpolarization (to activate the HCN channels) and for the initiation of APs in adjacent quiescent cells. For this reason, if undifferentiated cells are used, suppression of connexin function or suppression of load (as discussed hereinbefore) will not only reduce I_{K1} effects, but it will also reduce HCN activation. Suppression of connexin function or suppression of load (as discussed hereinbefore) is therefore not directly compatible with undifferentiated cells. However, undifferentiated cells provide an optimal tool to deliver I_{f} currents, possibly combined with increased intracellular cAMP, and, as such, they are preferably injected alone or in combination with the injection of gene therapy vectors or differentiated stem cells.
Differentiated stem cells, while being more difficult to culture and expand, are better capable of incorporating the various pacemaker properties. Various Ca²⁺ handling proteins (e.g., RyR2, SERCA2a,b,c and NCX-1) are efficiently upregulated in the differentiation process with 5'-azacytidine and TGF-β1 (TGF-beta 1), whereas incorporation of some of these genes in a gene therapy vector is hampered by their relatively large size. Biopacemaker properties are ultimately tailored by optimized differentiation towards a spontaneously active pacemaker phenotype, preferably in combination with additional gene transfer to increase HCN currents and/or increase intracellular cAMP. Additionally, TBX3 overexpression is used to stimulate differentiation into a nodal phenotype and prevent further differentiation into a more mature, working myocardium, phenotype.

The invention furthermore provides a vector or an isolated cell comprising a compound capable of providing and/or increasing a pacemaker current I_{f}, and a compound capable of diminishing electrical coupling between said cell and surrounding cells. A vector or an isolated cell comprising a compound capable of providing and/or increasing a pacemaker current I_{f} and a compound capable of reducing the inward rectifier current I_{K1} of said cell is also herewith provided. As explained before, said compound capable of providing and/or increasing a pacemaker current I_{f} preferably comprises a hyperpolarization-activated cyclic nucleotide-gated (HCN) channel or a nucleic acid sequence coding therefore. Other preferred compounds capable of providing and/or increasing a pacemaker current I_{f} are:
- a cAMP producing enzyme, an adenylate cyclase, adenylate cyclase-1, adenylate cyclase-8, a compound capable of increasing the amount and/or activity of a cAMP producing enzyme, a compound capable of reducing the amount and/or activity of an enzyme involved with cAMP breakdown, a phosphodiesterase with a diminished function as compared to wild type phosphodiesterase;
- a nucleic acid sequence encoding at least one of the abovementioned compounds; and
- an siRNA and/or antisense nucleotide sequence against a phosphodiesterase.

One embodiment of the present invention therefore provides a vector or an isolated cell comprising:
- a nucleic acid sequence or a functional equivalent thereof encoding a hyperpolarization-activated cyclic nucleotide-gated (HCN) channel, and
- an siRNA and/or antisense nucleotide sequence against a phosphodiesterase.

Another embodiment provides a vector or an isolated cell comprising:
- a nucleic acid sequence or a functional equivalent thereof encoding a hyperpolarization-activated cyclic nucleotide-gated (HCN) channel, and
- a nucleic acid sequence or a functional equivalent thereof encoding a compound selected from the group consisting of:
   a cAMP producing enzyme, an adenylate cyclase, adenylate cyclase-1, adenylate cyclase-8, a compound capable of increasing the amount and/or activity of a cAMP producing enzyme, a compound capable of reducing the amount and/or activity of an enzyme involved with cAMP breakdown, a phosphodiesterase with a diminished function as compared to wild type phosphodiesterase.

Preferred compounds capable of diminishing electrical coupling between a pacemaker cell and surrounding cells are, as described hereinbefore:
- a compound capable of reducing the amount and/or activity of gap junction proteins connecting said cell and surrounding cells, a gap junction protein with a diminished conductor capacity as compared to connexin 43, a gap junction protein with a diminished conductor capacity as compared to connexin 40, a compound capable of reducing the amount and/or activity of connexin 43 of said cell, a compound capable of reducing the amount and/or activity of connexin 40 of said cell, a connexin with a lower conductor capacity than the conductor capacity of connexin 43, a connexin with a lower conductor capacity than the conductor capacity of connexin 40; connexin 30.2, connexin 45, connexin 43Δ or a functional equivalent thereof, a transcription factor capable of reducing connexin 43 expression, a transcription factor capable of reducing connexin 40 expression, TBX3 or a functional equivalent thereof;
- a nucleic acid sequence encoding at least one of the abovementioned compounds; and
- an siRNA and/or antisense nucleotide sequence against connexin 43, an siRNA and/or antisense nucleotide sequence against connexin 40

Preferred embodiments of the present invention therefore provide a vector or an isolated cell comprising:
- a nucleic acid sequence or a functional equivalent thereof encoding a hyperpolarization-activated cyclic nucleotide-gated (HCN) channel, and
- an siRNA against connexin 43, and/or an antisense nucleotide sequence against connexin 43, and/or an siRNA against connexin 40, and/or an antisense nucleotide sequence against connexin 40, and/or a nucleic acid sequence or a functional equivalent thereof encoding a compound selected from the group consisting of:
   a compound capable of reducing the amount and/or activity of gap junction proteins connecting said cell and surrounding cells, a gap junction protein with a diminished conductor capacity as compared to connexin 43, a gap junction protein with a diminished conductor capacity as compared to connexin 40, a compound capable of reducing the amount and/or activity of connexin 43 of said cell, a compound capable of reducing the amount and/or activity of connexin 40 of said cell, a connexin with a lower conductor capacity than the conductor capacity of connexin 43, a connexin with a lower conductor capacity than the conductor capacity of connexin 40, connexin 30.2, connexin 45, connexin 43Δ or a functional equivalent thereof, a transcription factor capable of reducing connexin 43 expression, a transcription factor capable of reducing connexin 40 expression, TBX3 or a functional equivalent thereof.

Furthermore, preferred compounds capable of reducing the inward rectifier current I_{K1} of a pacemaker cell are, as described hereinbefore:
- an inwardly-rectifying potassium channel with a diminished function as compared to the same kind of inwardly-rectifying potassium channel in a wild type form, and a Kir2.1 channel or a functional equivalent thereof;
- a nucleic acid sequence encoding at least one of the abovementioned compounds; and
- an siRNA and/or antisense nucleotide sequence against an inwardly-rectifying channel.

A preferred embodiment of the present invention therefore provides a vector or an isolated cell comprising:
- a nucleic acid sequence or a functional equivalent thereof encoding a hyperpolarization-activated cyclic nucleotide-gated (HCN) channel, and
- an siRNA and/or antisense nucleotide sequence against an inwardly-rectifying channel.

Another preferred embodiment provides a vector or an isolated cell comprising:
- a nucleic acid sequence or a functional equivalent thereof encoding a hyperpolarization-activated cyclic nucleotide-gated (HCN) channel, and
- a nucleic acid sequence or a functional equivalent thereof encoding a compound selected from the group consisting of:
   an inwardly-rectifying potassium channel with a diminished function as compared to the same kind of inwardly-rectifying potassium channel in a wild type form, and a Kir2.1 channel or a functional equivalent thereof.

In one preferred embodiment a cell according to the present invention comprises a myocardial cell. A method or a cell according to the invention, wherein said cell comprises a myocardial cell, is therefore also provided. In one embodiment said cell comprises a cardiac stem cell or cardiac progenitor cell. These embodiments are particularly suitable for cardiovascular applications.

A method according to the invention is particularly suitable for treating a subject suffering from, or at risk of suffering from, a disorder associated with impaired function of a cell with a spontaneous electrical activity. Restoring or improving spontaneous cellular electrical activity with a method according to the invention, and/or providing a cell with a spontaneous electrical activity with a method according to the invention, results in alleviation of the symptoms of said disease and/or at least partial treatment of said disease. Further provided is therefore a method for treating a subject suffering from, or at risk of suffering from, a disorder associated with impaired function of a cell with a spontaneous electrical activity, the method comprising:
- providing a cell of said subject with spontaneous electrical activity with a method according to the invention, and/or
- increasing the depolarization rate of a cell of said subject with a method according to the invention, and/or
- administering to said subject a therapeutic amount of a vector and/or a cell according to the invention.

In a preferred embodiment said disorder associated with impaired function of a cell with a spontaneous electrical activity is a cardiovascular disorder. A biopacemaker is preferably provided with a method according to the present invention. Further provided is therefore a method for treating a subject suffering from, or at risk of suffering from, a cardiovascular disorder, the method comprising:
- providing a myocardial cell of said subject with spontaneous electrical activity with a method according to the invention, and/or
- increasing the depolarization rate of a myocardial cell of said subject with a method according to the invention, and/or
- administering to said subject a therapeutic amount of a vector and/or a cell according to the invention. Said vector and/or cell is preferably administered to an atrium or a ventricle of the heart of said subject.
   In one preferred embodiment said cardiovascular disorder comprises a cardiac conduction disorder, preferably sick sinus syndrome and/or AV nodal block.

Dose ranges of compounds, nucleic acid sequences, vectors and cells according to the invention to be used in the therapeutic applications as described herein are preferably designed on the basis of rising dose studies in the clinic in clinical trials for which rigorous protocol requirements exist. Typically, a dose of 0.1 - 3 ml 1*10⁸ - 1*10¹⁰ TU/ml is used with lentiviral vectors. In one embodiment a compound, nucleic acid sequence and/or cell according to the invention is combined with a pharmaceutically acceptable excipient, stabilizer, activator, carrier, permeator, propellant, desinfectant, diluent and/or preservative. Suitable excipients are commonly known in the art of pharmaceutical formulation and may be readily found and applied by the skilled artisan. A non-limiting example of a suitable excipient for instance comprises PBS.
A subject (preferably a human being) is provided with an effective amount of a compound, nucleic acid sequence, vector and/or cell according to the invention via any suitable route of administration. For instance, a (vector comprising a) nucleic acid is injected into cells of interest of a subject, for instance into myocardial cells of a subject. Preferably at least one parameter indicative of a disorder associated with impaired function of a cell with a spontaneous electrical activity, for instance a cardiovascular disorder, is determined before and after administration of a compound, nucleic acid sequence, vector and/or cell according to the invention, allowing determining whether or not treatment is successful. If desired, administration of further doses is repeated as often as necessary, preferably until the above mentioned at least one parameter is considered to be acceptable. One example of a suitable parameter is the heart rate at rest and during exercise and the presence or absence of arrhythmias, complaints or signs/symptoms of impaired cardiovascular function (e.g., reduced exercise capacity, heart failure, dizziness, syncope).

Another aspect of the present invention provides a device for increasing the depolarization rate of a cell or a group of cells having spontaneous electrical activity, and/or for providing a cell or a group of cells with spontaneous electrical activity, said device comprising:
- means for providing a cell with a compound capable of providing and/or increasing a pacemaker current I_{f}, and
- means for diminishing electrical coupling between said cell or group of cells and surrounding cells.
   Such device is particularly suitable for performing a method according to the invention, wherein a cell or a group of cells is provided with a compound capable of providing and/or increasing a pacemaker current I_{f}, and wherein electrical coupling between said cell(s) and surrounding cells is diminished. This provides better results as compared to conventional methods. A device according to the invention preferably comprises a catheter. Said means for providing a cell with a compound capable of providing and/or increasing a pacemaker current I_{f}, and said means for diminishing electrical coupling between said cell and surrounding cells are preferably different from each other. In one embodiment, said means for diminishing electrical coupling between said cell and surrounding cells comprises a heating element. Said heating element preferably comprises an element for radiofrequency ablation, as described herein before. In another preferred embodiment, said means for diminishing electrical coupling between said cell and surrounding cells comprises a cooling element, preferably an element for cryo ablation. Said means for providing a cell with a compound capable of providing and/or increasing a pacemaker current I_{f} preferably comprises an element for injection of a nucleic acid sequence.

In a particularly preferred embodiment, a device according to the present invention comprises a catheter comprising a heating element or a cooling element, as well as an element for injection of a nucleic acid sequence. Such catheter is preferably used in a method according to the invention wherein a cell or a group of cells is provided with a compound capable of providing and/or increasing a pacemaker current I_{f}, and wherein electrical coupling between said cell(s) and surrounding cells is diminished.

A device according to the invention preferably comprises a heating element or a cooling element with a shape which enables limitation of electrical connections between a pacemaker cell and surrounding tissue in at least one direction. After use of such device electrical connections between a pacemaker cell and surrounding tissue are primarily present in one or several directions, whereas electrical impulses to at least one other direction are diminished. Preferably, electrical impulses to at least one other direction are blocked. This allows regulation of impulse conduction into one or several desired directions. A device according to the invention preferably has a shape in which electrical connections to the surrounding tissues are only present in a limited amount of directions with respect to the plane that runs parallel to the heart surface. This means that impulse conduction is limited and/or blocked in at least one direction. Further provided is therefore a device according to the invention, which has a shape that allows for diminishing, preferably blocking, electrical connections between said cell or group of cells and the surrounding tissues in at least one direction. As explained before, the electrical coupling between a pacemaker cell and surrounding cells is preferably diminished such that the electrical impulse of a firing pacemaker cell will be conducted into a certain direction. This is for instance performed by providing a conductor barrier which partly surrounds said pacemaker cell or group of pacemaker cells. A non-limiting example thereof is schematically depicted in Figure 1:
an electrical impulse of a firing pacemaker cell is conducted to a certain direction because other directions are blocked by a barrier (for instance fibrotic cells).

A method according to the invention involves the use of a compound capable of providing and/or increasing a pacemaker current I_{f}, together with a compound capable of diminishing electrical coupling between said cell and surrounding cells and/or a compound capable of reducing the inward rectifier current I_{K1} of said cell. Such combination of compounds is suitable for therapeutic purposes in order to counteract a disorder associated with impaired function of a cell with spontaneous electrical activity. Further provided is therefore a combination of:
- a compound capable of providing and/or increasing a pacemaker current I_{f}, and
- a compound capable of diminishing electrical coupling between said cell and surrounding cells and/or a compound capable of reducing the inward rectifier current I_{K1} of said cell, for use as a medicament.

Also provided is a use of:
- a compound capable of providing and/or increasing a pacemaker current I_{f}, and
- a compound capable of diminishing electrical coupling between said cell and surrounding cells and/or a compound capable of reducing the inward rectifier current I_{K1} of said cell, for the preparation of a medicament for preventing or counteracting a disorder associated with impaired function of a cell with spontaneous electrical activity.

Said combination is preferably used for the preparation of a medicament against as a cardiovascular disorder. One embodiment thus provides a use of:
- a compound capable of providing and/or increasing a pacemaker current I_{f}, and
- a compound capable of diminishing electrical coupling between said cell and surrounding cells and/or a compound capable of reducing the inward rectifier current I_{K1} of said cell, for the preparation of a medicament for preventing or counteracting a cardiovascular disorder.

In one preferred embodiment said compound capable of diminishing electrical coupling between said cell and surrounding cells comprises a device according to the invention, as described herein before. Most preferably, a catheter comprising a heating element or a cooling element, as well as an element for injection of a nucleic acid sequence, is used.

In yet another preferred embodiment a combination or use according to the invention is provided, wherein said compound capable of diminishing electrical coupling between said cell and surrounding cells comprises an siRNA and/or antisense nucleotide sequence against connexin 43 and/or an siRNA and/or antisense nucleotide sequence against connexin 40 and/or a nucleic acid sequence encoding a compound selected from the group consisting of:
a compound capable of reducing the amount and/or activity of gap junction proteins connecting said cell and surrounding cells, a gap junction protein with a diminished conductor capacity as compared to connexin 43, a gap junction protein with a diminished conductor capacity as compared to connexin 40, a compound capable of reducing the amount and/or activity of connexin 43 of said cell, a compound capable of reducing the amount and/or activity of connexin 40 of said cell, a connexin with a lower conductor capacity than the conductor capacity of connexin 43, a connexin with a lower conductor capacity than the conductor capacity of connexin 40, connexin 30.2, connexin 45, connexin 43Δ or a functional equivalent thereof, a transcription factor capable of reducing connexin 43 expression, a transcription factor capable of reducing connexin 40 expression and TBX3 or a functional equivalent thereof.

Additionally, or alternatively, a combination or use according to the invention is provided wherein said compound capable of providing and/or increasing a pacemaker current I_{f} comprises an siRNA and/or antisense nucleotide sequence against a phosphodiesterase and/or a nucleic acid sequence encoding a compound selected from the group consisting of: a cAMP producing enzyme, an adenylate cyclase, adenylate cyclase-1, adenylate cyclase-8, a compound capable of increasing the amount and/or activity of a cAMP producing enzyme, a compound capable of reducing the amount and/or activity of an enzyme involved with cAMP breakdown, and a phosphodiesterase with a diminished function as compared to wild type phosphodiesterase.

Additionally, or alternatively, a combination or use according to the invention is provided wherein said compound capable of reducing the inward rectifier current I_{K1} of said cell comprises an siRNA and/or antisense nucleotide sequence against an inwardly-rectifying potassium channel and/or a nucleic acid sequence encoding a compound selected from the group consisting of: an inwardly-rectifying potassium channel with a diminished function as compared to the same kind of inwardly-rectifying potassium channel in a wild type form, and a Kir2.1 channel or a functional equivalent thereof.

A pharmaceutical composition, comprising a vector and/or a cell according to the invention, is also provided herein. Said composition optionally comprises a pharmaceutically acceptable excipient, stabilizer, activator, carrier, propellant, desinfectant, diluent and/or preservative. Suitable excipients are commonly known in the art of pharmaceutical formulation and may be readily found and applied by the skilled artisan

The invention is further explained in the following examples. These examples do not limit the scope of the invention, but merely serve to clarify the invention.

### Examples

### Prior Art Biopacemakers

Research on biological pacemakers has so far mainly focused on proof-of-principle concepts. None of these concepts provided stable function at an acceptable heart rate.

### Improving biopacemakers

To develop a clinically relevant biological pacemaker, stable long-term function at a physiological heart rate and incorporation of autonomic modulation are crucial. We started our research with lentiviral vectors in an effort to ensure long-term overexpression of HCN4. Novel strategies to improve this biopacemaker are also developed. These improvements center around two concepts: (1) improving impulse formation to enhance basal pacing rate in combination with tailored autonomic responsiveness and (2) protecting impulse formation to stabilize basal pacing rate. To unravel the most important contributors to biopacemaker function, we employ different strategies in parallel.

### Materials and methods

### Construction and production of lentiviral vectors

The cDNAs for human HCN4 (Alexander Scholten, Institut für Biologische Informationsverarbeitung, Forschungszentrum Jülich, Jülich, Germany) and rat Cx43_130 to 136 deletion mutant (Vladimir Krutovskikh, International Agency for Research on Cancer, Lyon, France) were sub-cloned into the lentiviral vector plasmid, pRRL-cPPT-CMV-PRE-SIN. [24] These vectors were designated LV-HCN4 and LV-Cx43Δ. A control vector in which the CMV promoter drives GFP expression (LV-GFP) was described earlier [24]. Additional bicistronic vector plasmids were constructed with HCN4 and TBX3. In this vector, gene expression is controlled by a CMV promoter and transgene expression is linked to GFP expression by an internal ribosome entry site (IRES) from the encephalomyocarditis virus (EMCV). These vectors were designated LV-HCN4-GFP and LV-TBX3-GFP, respectively. Lentiviral vectors were generated by cotransfection of HEK293T cells, concentrated and titrated as described previously [25]. LV-HCN4 and LV-Cx43Δ was generated similarly and titrated by detecting transgene expression on transduced HeLa cells with immunohistochemistry.

### Cell isolation and culture of neonatal rat ventricular cardiac myocytes

Animal experiments were performed in accordance with the Guide for the Care and Use of Laboratory Animals published by the National Institute of Health (NIH Publication No. 85-23, revised 1996), and approved by the institutional committee for animal experiments.

Six neonatal rats were sacrificed in one procedure as described previously [26]. Briefly, rats were decapitated after which a cardiotomy was performed. The atria were removed and the ventricles were minced. Tissue fragments were washed, using a Hanks' balanced salt solution (HBSS) without Ca²⁺ and Mg²⁺ supplemented with 20 units/100ml penicillin and 20 µg/100ml streptomycin. Five to six dissociations were performed for 15 minutes at 36.5° C. The dissociations were performed using HBSS without Ca²⁺ and Mg²⁺ containing 20 units/100ml penicillin, 20 µg/100ml streptomycin, 0.2% trypsin and 60µg/ml pancreatin. The obtained dissociation solutions were centrifuged and cell pellets were resuspended in culture medium.
The neonatal rat ventricular myocytes were cultured in M199 containing (mM): 137 NaCl, 5.4 KCl, 1.3 CaCl₂, 0.8 MgSO₄, 4.2 NaHCO₃, 0.5 KH₂PO₄, 0.3 Na₂HPO₄, and supplemented with 20 units/100ml penicillin, 20 µg/100ml streptomycin, 2 µg/100ml vitamin B₁₂ and either 5% or 10% neonatal calf serum (NCS), 10% NCS was used only on the first day of culturing the cells. These cells were cultured on collagen coated glass at 37° C in 5% CO₂.

### Cardiac progenitor cells isolation and differentiation

Cardiac myocyte progenitor cells were isolated from human fetal hearts obtained after elected abortion with prior informed consent and approval of the ethical committee of the University Medical Center Utrecht. Hearts were isolated and perfused using a Langendorff perfusion setup. After digestion with collagenase and protease, CMPCs were isolated from the cardiac cell suspension using magnetic beads coated with a Sca-1 antibody. Cells were cultured on 0.1% gelatin coated material, using SP++ medium (EBM-2 with EGM-2 additives, mixed 1:3 with M199) supplemented with 10% FCS (Gibco), 10 ng/ml basic Fibroblast growth factor (bFGF), 5 ng/ml epithelial growth factor (EGF), 5 ng/ml insuline like growth factor (IGF-1) and 5 ng/ml hepatocyte growth factor (HGF). CMPCs were differentiated in Iscove's Modified Dulbecco's Medium /Ham's F-12 (1:1) (Gibco) supplemented with L-Glutamine (Gibco), 2% horse serum, non-essential amino acids, Insulin-Transferrin-Selenium supplement, and 10-4 M ascorbic acid (Sigma). First, CMPCs were exposed to 5 µM 5'-azacytidine for three days, followed with 1 ng/mL TGF-β 1 every three days. For electrophysiological experiments, differentiated cultures were dissociated using collagenase and replated on gelatin coated coverslips in densities ranging from single cells to monolayers.

### Single cell transduction and electrophysiological recordings

Neonatal rat cardiac myocytes and CMPCs were transduced with LV-HCN4-GFP, TBX3-GFP and LV-GFP at a MOI of 0.1. Single electrophysiological cell experiments were performed 7-10 days and 2 months after transducing NRCMs and CMPCs, respectively. Myocytes were trypsinized during 30 seconds to prepare them for patch-clamping. By this procedure, cardiac myocytes lost their cell-to-cell connections, became less flattened (which facilitated the use of glass micropipettes), but remained attached to the coverslip.

Action potentials, I_{f} and membrane currents were recorded at 36±0.2° using the perforated patch-clamp technique (Axopatch 200B Clamp amplifier, Axon Instruments Inc.). Signals were low-pass filtered (cut-off frequency: 5-kHz) and digitized at 5-kHz. Series resistance was compensated by ≥80%, and potentials were corrected for the estimated 15-mV change in liquid-junction potential. For voltage control, data acquisition, and analysis, custom-made software was used. Superfusion solution contained (mM): 140 NaCl, 5.4 KCl, 1.8 CaCl₂, 1.0 MgCl₂, 5.5 glucose, 5 HEPES; pH 7.4 (NaOH). Pipettes (2-3 M□, borosilicate glass) were filled with solution containing (mM): 125 K-gluc, 20 KCl, 5 NaCl, 0.22 amphotericin-B, 10 HEPES; pH 7.2 (KOH).

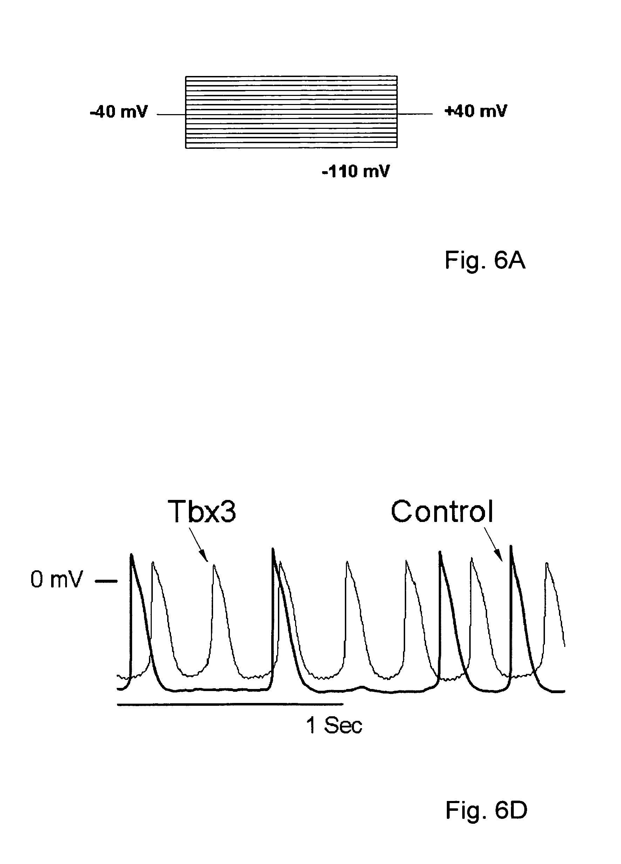

If was characterized using custom voltage-clamp protocols modified from those published previously [14,27]. For current-voltage (I-V) relationships and activation properties, If was measured as Cs⁺ sensitive (5 mM) current during 6-s hyperpolarizing steps (range -30 to -110 mV) from a holding potential of -30 mV. The hyperpolarizing step was followed by an 8-s step to -110 mV to record tail current, then a 0.5-s pulse to 40 mV to ensure full deactivation Tail current, plotted against test voltage, provided the activation-voltage relation; the latter was normalized by maximum conductance and fitted with the Boltzmann function I/Iₘₐₓ=A/{1.0+exp[(V_{1/2}—V)/k]} to determine the half-maximum activation voltage (V_{1/2}) and slope factor (k).
Net membrane current was characterized by 500-ms hyper-and depolarizing voltage-clamp steps from a holding potential of -40 mV every 2 s (for protocol, see Figure 6A) Membrane currents were normalized to cell size.

### Focal transduction and optical mapping

Monolayers of NRCMs were inspected, and those with defects or nonbeating cultures were rejected before transduction. Cells were transduced with lentiviral vectors, 4 days after culture and optical mapping performed 4 days after transduction. Focal transduction of the central area in the monolayer was obtained using lentiviral vectors complexed to magnetic nanoparticles (System Biosciences). These complexes were subsequently injected just above the monolayer (∼1mm), above a strong magnetic field.

Coverslips were stained with 50 µmol/L di-8-ANEPPS (Molecular Probes) for 15 minutes. Optical recordings were made in a custom-made setup. Excitation light was delivered by a cyan (505 nm) high power Light Emitting Diode (LED) filtered by a 505 - 30 nm band-pass filter. Emission fluorescence was high-pass filtered (600 nm) and measured with a photodiode array (PDA; Hamamatsu C4675-102). Data acquisition was performed with modified ActiveTwo system without the input-amplifiers (BioSemi); data were analyzed using custom made software based on Matlab (Mathworks) [30]. After acquisition, data were digital filtered.

### In vivo tamoxifen inducible TBX3 overexpression

Animal care was in accordance with national and institutional guidelines.

The *TBX3*^{Cre} allele has been previously described [31]. For age determination of the embryos, couples were put together overnight when the female was in estrus. On the next day, the female was inspected for a vaginal plug and the animals were separated. Noon was considered ED 0.5. Genomic DNA prepared from amnion or tail biopsies was used for genotyping by PCR, using primers specific for Cre and the wild-type allele. Animal care was in accordance with national and institutional guidelines.
CAG-CAT-TBX3 [31,32], MerCreMer [33] and Z/EG [34]transgenic mice have been described previously. The transgenic mice were identified by PCR analysis using primers specific for *CAT, Cre* and *GFP* genes. MerCreMer (MCM) transgenic mice were bred with CAG-CAT-TBX3 (CT3) / Z/EG double transgenic mice to generate MCM-CT3, MCM-Z/EG double or MCM-CT3-Z/EG triple transgenic mice. These mice have no phenotype. Upon administration of tamoxifen (Sigma T5648, 1mg, intraperitoneal injection, 4 days) MerCreMer is activated and causes recombination according to the Cre-loxP system. CAT and lacZ are recombined out of the CT3 and Z/EG constructs respectively, which results in TBX3 and EGFP expression in all cardiomyocytes, because the MerCreMer gene is driven by a heart specific promoter (α-MHC). This way TBX3 over expression can be studied in adult mice. After 4 days of tamoxifen administration mice were sacrificed and the hearts were isolated. Expression of EGFP as a positive control of successful recombination and a marker for leakage of the system in non-tamoxifen treated animals was evaluated by fluorescent microscopy. The left atrial appendage and the apex were separated from the rest of the heart and all parts of the heart were snap frozen in liquid nitrogen quickly.
Total RNA was isolated from apex and left atrial appendices using the Nucleospin Kit (Machery-Nagel) according to the manufacturer's protocol. cDNA was made by reverse-transcription of 300 ng of total RNA using the SuperScript II system (Invitrogen). Expression of genes was assayed with quantitative real-time PCR using the Lightcycler 480 (Roche). qPCR data were analyzed with LinRegPCR (Ramakers et al, 2003) to determine the PCR efficiency values per sample. Starting concentrations were calculated per sample using the mean PCR efficiency per amplicon and the individual CT-values [35]. Gene expression data are presented normalized for GAPDH expression.
Results are expressed as mean ± SEM. Data are considered significantly different if *P* < 0.05. unpaired t-test

### Example 1

### Increasing intracellular cAMP

Basal cAMP levels are almost tenfold higher in SA node cells as compared to atrial and ventricular myocytes. The increase in intracellular cAMP both stimulates Ca²⁺ handling proteins and shifts the If activation curve towards more depolarized potentials. Both changes increase beating rates. Increased intracellular cAMP levels are for instance achieved by overexpressing cAMP producing enzymes (adenylate cyclase, AC) or reducing the expression or function of enzymes responsible for cAMP breakdown (phosphodiesterases, PDEs). In particular, PDE4 appears to be involved in β-adrenergic signaling of both sarcolemmal ion channels (e.g., HCN) and sarcoplasmic reticulum (SR) Ca²⁺ handling proteins (e.g., SERCA). Genetic suppression of PDE activity (for instance with siRNAs or PI3K_{Y}; an upstream modulator of PDE activity) are therefore considered important strategies to improve biopacemaker function.

### Results

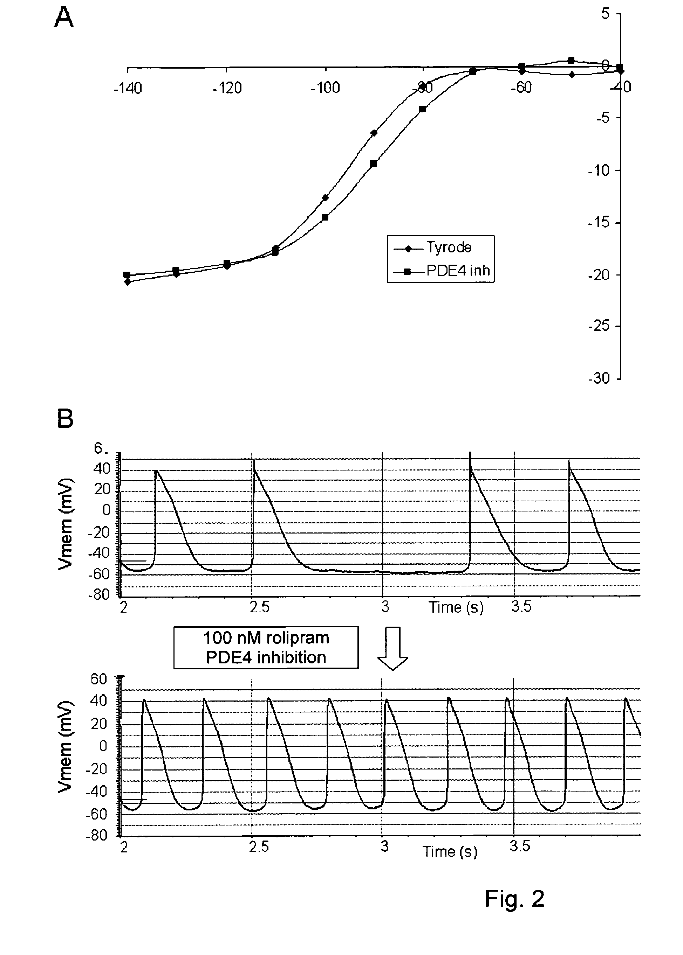

■ PDE4 inhibition using rolipram in combination with HCN4 overexpression demonstrates a rightward shift in the If activation curve in NRCMs (see Figure 2A)
■ PDE4 inhibition with rolipram in itself also demonstrates a remarkable increase in spontaneous activity in single NRCMs (Figure 2B)

### Example 2

### Fine tuning of nodal properties in engineered stem cells

In addition to the various gene therapy strategies described hereinbefore, stem cells provide an alternative delivery platform or pacemaker source, especially when upregulation or downregulation of *multiple* genes is required to improve overall pacemaker function. In our effort to improve impulse formation, we described strategies to increase HCN current and to increase intracellular cAMP. These strategies can be incorporated and further developed in engineered stem cells. We therefore designed a third strategy that combines *ex vivo* lentiviral gene transfer using undifferentiated stem cells (human cardiac myocyte progenitor cells, hCMPCs, or human mesenchymal stem cells, hMSCs) or differentiated stem cells (hCMPCs). *Ex vivo* lentiviral gene transfer provides an efficient strategy to introduce multiple genes which allows for the creation of a homogeneous stem cell population with optimal pacemaker characteristics. The risk of immunogenic rejection is maximally reduced by the use of autologous cells.

Undifferentiated stem cells are easier to culture and to expand, and they may also be immunoprivileged. However, these cells only function as a delivery system (of an inward pacemaker current). These cells lack the complete set of ion channels involved in membrane hyperpolarization and generation of cardiac action potentials (APs). Connexin proteins, importantly involved in electrical coupling and cell-to-cell transmission of the electrical impulse, are therefore important in both achieving the required membrane hyperpolarization (to activate the HCN channels) and for the initiation of APs in adjacent quiescent cells. For this reason, suppression of connexin function or suppression of load will not only reduce I_{K1} effects, but it will also reduce HCN activation and is therefore not directly compatible with undifferentiated cells. However, undifferentiated cells provide an optimal tool to deliver I_{f} currents and, possibly combined with increased intracellular cAMP, as such, they are preferably combined with the injection of gene therapy vectors or differentiated stem cells.

We have transduced undifferentiated hCMPCs by lentiviral vectors.

### Results

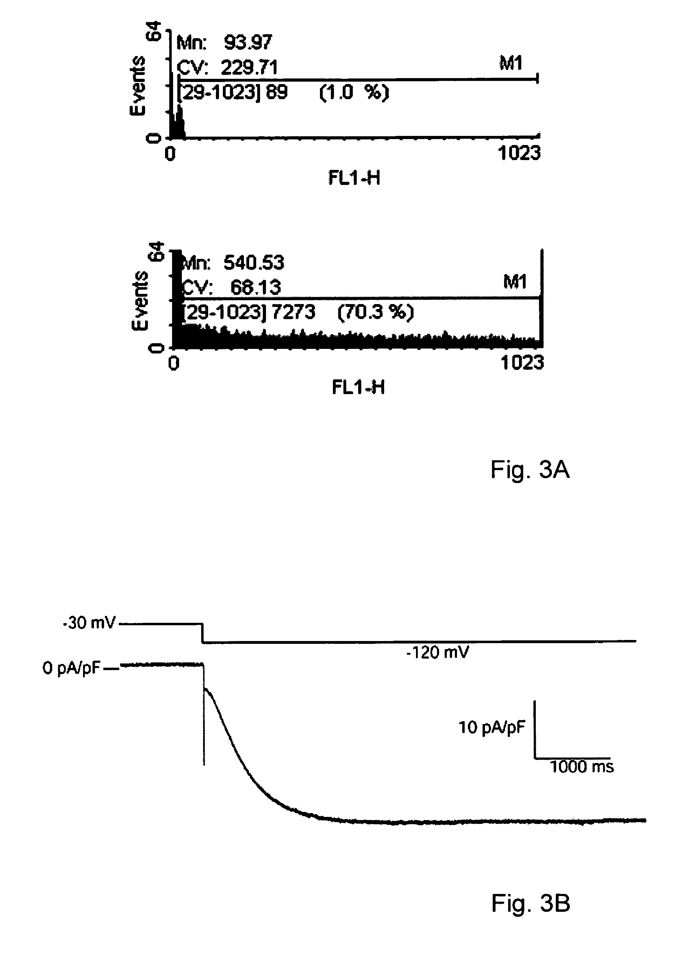

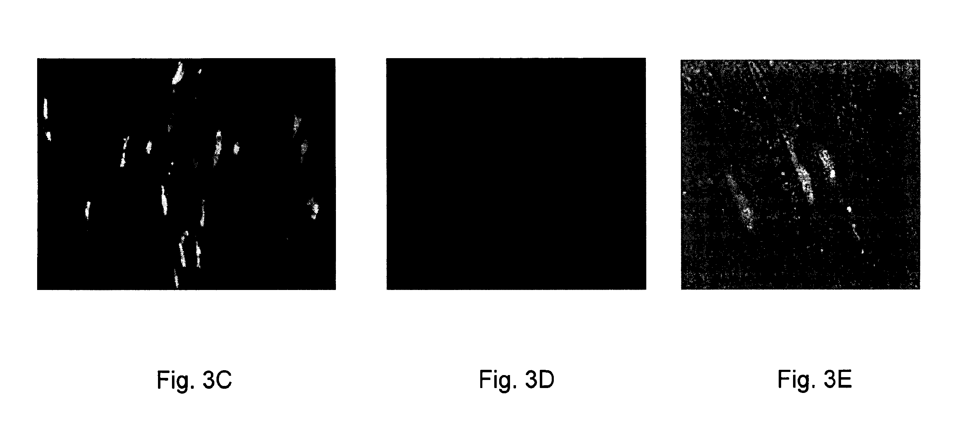

■ Undifferentiated hCMPCs are efficiently transduced by lentiviral vecors; at a multiplicity of infection (MOI) of 100 approximately 70% of the cells were transduced (Figure 3A).
■ Undifferentiated hCMPCs transduced with LV-HCN4-GFP demonstrated stable If expression for more than 2 months (Figure 3B).
■ Basic cell morphology remains intact after lentiviral overexpression of GFP, HCN4 or TBX3 (Figure 3C, D, E).

### Example 3

### Protecting impulse formation to stabilize the biopacemaker rate: impulse protecting genes and procedures

Currently constructed biopacemakers using engineered HCN mutants or very large amounts of HCN overexpressing hMSCs have failed to increase the biopacemaker frequency within the physiological range. This probably results from an intensified response to parasympathetic stimulation during rest, a failure to drive, or a combination of both. In the previous examples we provided solutions to problems that could result from an intensified response to parasympathetic stimulation. Here, we describe novel biopacemaker concepts to protect impulse formation and thereby provide solutions for problems that center around load-mismatch and a failure to drive. Protecting the biopacemaker area by partial electrical uncoupling, reducing the electrical load, and/or physical uncoupling stabilizes function and subsequently reduces the required size of the biopacmaker region.

Partial uncoupling is for instance achieved by overexpression of certain connexin isoforms (depending on the target region, e.g., Cx30.2, Cx40 or Cx45) or suppression of Cx43 (with dominant negative constructs [e.g., Cx43Δ], siRNA or transcription factor overexpression, e.g., TBX3).

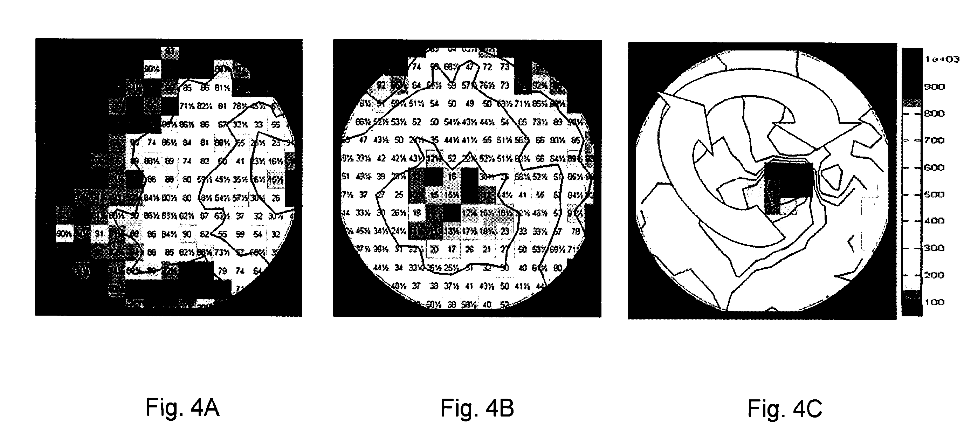

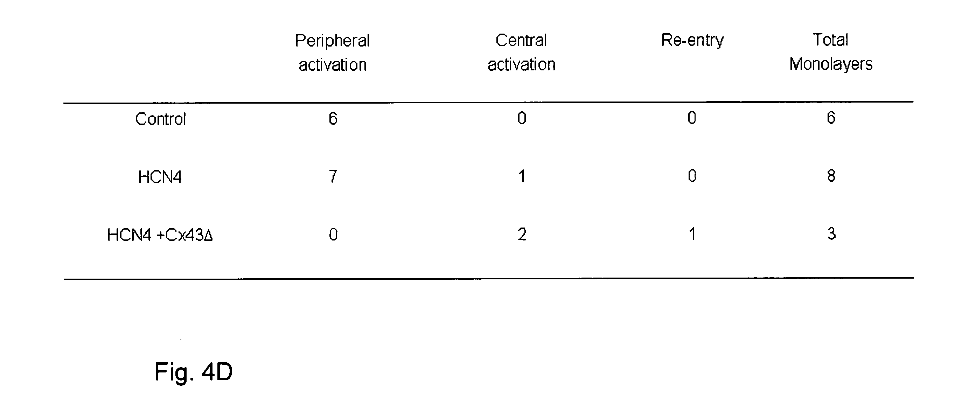

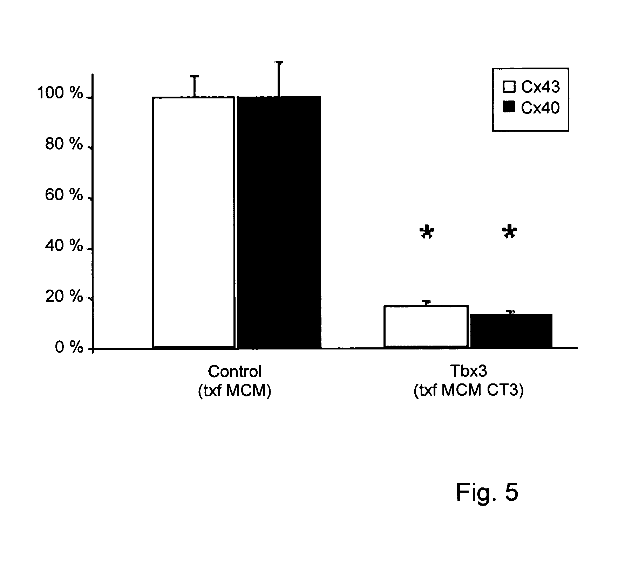

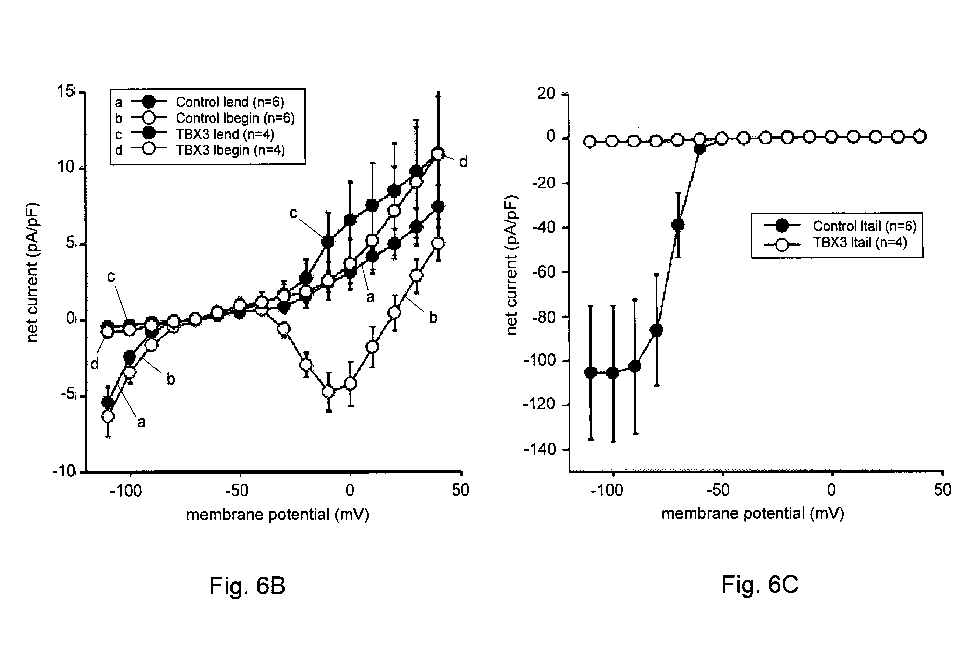

**Results**
■ Monolayers engineered with central HCN4 expression demonstrate improved impulse formation in combination with Cx43Δ (Figure. 4)
■ Partial uncoupling with Cx43Δ probably also increases the susceptibility to re-entry arrhythmias. (Figure 4C)
■ Inducible TBX3 expression initiates down regulation of both connexin 43 and connexin 40 in adult atrial myocardium (Figure 5)
■ In TBX3 overexpressing NRCMs, we observed a reduced instantaneous current in the voltage range of Ic_{a,L} activation and a reduced steady-state current in the voltage range of I_{K1} activation (Figure 6B)
■ In control NRCMs, we observed a large transient inward current by stepping back from very negative potential with characteristics of the Na⁺ current. This current was absent in TBX3 overexpression cells (Figure 6C)
■ TBX3 overexpressing NRCMs adopt hallmark features of nodal cells, possibly due to a reduced 'background' K⁺ current, I_{K1} (Figure 6D).

### Brief description of the drawings

**Figure 1****.** Schematic diagram of a non-limiting example of biopacemaker impulse formation combined with impulse protection.
**Figure 2****.** Improved impulse formation by PDE4 inhibition. A, Neonatal rat cardiac myocytes overexpressing HCN4 demonstrate a shift in the If activation curve with PDE4 inhibition by 100 nM rolipram. B, Increased spontaneous activity in control neonatal rat cardiac myocytes exposed to PDE4 inhibition by 100 nM rolipram.
**Figure 3****.** Cardiac progenitor cells transduced with lentiviral vectors. A, FACS analyses of control (upper panel) and LV-GFP transduced (lower panel) CMPCs. A transduction efficiency of nearly 70% was obtained with a multiplicity of infection of 100. B, Typical If trace of a LV-HCN4-GFP transduced CPC, 2 months after transduction. Fluorescence microscopy of LV-GFP transduced CMPCs (C), LV-HCN4-GFP transduced CMPCs (D) and LV-TBX3-GFP transduced CMPCs (E).
**Figure 4****.** Improved central activation by partial uncoupling. Typical examples activation maps of monolayers demonstrating peripheral (A), central (B) and re-entry (C) activation. D, Combined data are summarized in the Table of this figure.
**Figure 5****.** Inducible TBX3 overexpression in adult myocardium. Quantitative PCR analysis on samples of 6 left atria of 3 tamoxifen-treated MCM-CT3 mice and 3 tamoxifen-treated MCM mice as controls. Connexin 43 and 40 were down regulated by 17% and 13% (p-value < 0.01), respectively.
**Figure 6****.** *In vitro* characterization of lentiviral TBX3 overexpression. A, Pulse protocol, Net current was measured at the beginning and the end of hyper- and depolarizing voltage clamp step (panel B) and by stepping back to the holding potential of -40 mV (panel C). B,, Average I-V relationship of net membrane currents at begin (I_{begin}) and end (I_{end}) of the voltage steps.TBX3 overexpressing cells as well as controls do not demonstrate a larger I_{end} compared to I_{begin}, indicative for absence of a large I_{f}. In TBX3 overexpressing cells, we observed a reduced instantaneous current in the voltage range of I_{Ca,L} activation and a reduced steady-state current in the voltage range of I_{K1} activation. C, Absence of current activation in TBX3 overexpressing cells after voltage clamp steps demonstrates strongly reduced I_{Na}. D, TBX3 overexpressing cells demonstrate increased firing frequency, less negative MDP and slower upstroke velocity, characteristics of nodal cells.

### References

1 Qu J, Plotnikov AN, Danilo P, Jr., Shlapakova I, Cohen IS, Robinson RB et al. Expression and function of a biological pacemaker in canine heart. Circulation 2003: 107(8):1106-1109.
2 Miake J, Marbán E, Nuss HB. Biological pacemaker created by gene transfer. Nature 2002: 419(6903):132-133.
3 Edelberg JM, Huang DT, Josephson ME, Rosenberg RD. Molecular enhancement of porcine cardiac chronotropy. Heart 2001: 86(5):559-562.
4 Potapova I, Plotnikov A, Lu Z, Danilo P, Jr., Valiunas V, Qu J et al. Human mesenchymal stem cells as a gene delivery system to create cardiac pacemakers. Circ Res 2004: 94(7):952-959.
5 Plotnikov AN, Sosunov EA, Qu J, Shlapakova IN, Anyukhovsky EP, Liu L et al. Biological pacemaker implanted in canine left bundle branch provides ventricular escape rhythms that have physiologically acceptable rates. Circulation 2004: 109(4):506-512.
6 Kehat I, Khimovich L, Caspi O, Gepstein A, Shofti R, Arbel G et al. Electromechanical integration of cardiomyocytes derived from human embryonic stem cells. Nat Biotechnol 2004: 22(10):1282-1289.
7 Plotnikov AN, Shlapakova IN, Kryukova Y, Bucchi A, Pan ZM, Danilo PJ et al. Comparison of mHCN2 and mHCN2-E324A genes as biological pacemakers. Circulation 2005: 112(17):U180.
8 Xue T, Cho HC, Akar FG, Tsang SY, Jones SP, Marban E et al. Functional integration of electrically active cardiac derivatives from genetically engineered human embryonic stem cells with quiescent recipient ventricular cardiomyocytes: insights into the development of cell-based pacemakers. Circulation 2005: 111(1):11-20.
9 Bucchi A, Plotnikov AN, Shlapakova I, Danilo P, Jr., Kryukova Y, Qu J et al. Wild-type and mutant HCN channels in a tandem biological-electronic cardiac pacemaker. Circulation 2006: 114(10):992-999.
10 Tse HF, Xue T, Lau CP, Siu CW, Wang K, Zhang QY et al. Bioartificial sinus node constructed via in vivo gene transfer of an engineered pacemaker HCN Channel reduces the dependence on electronic pacemaker in a sick-sinus syndrome model. Circulation 2006: 114(10):1000-1011.
11 Kashiwakura Y, Cho HC, Barth AS, Azene E, Marbán E. Gene transfer of a synthetic pacemaker channel into the heart: a novel strategy for biological pacing. Circulation 2006: 114(16):1682-1686.
12 Cho HC, Kashiwakura Y, Marban E. Creation of a biological pacemaker by cell fusion. Circ Res 2007: 100(8):1112-1115.
13 Plotnikov AN, Shlapakova I, Szabolcs MJ, Danilo P, Jr., Lorell BH, Potapova IA et al. Xenografted Adult Human Mesenchymal Stem Cells Provide a Platform for Sustained Biological Pacemaker Function in Canine Heart. Circulation 2007.
14 Qu J, Barbuti A, Protas L, Santoro B, Cohen IS, Robinson RB. HCN2 overexpression in newborn and adult ventricular myocytes: distinct effects on gating and excitability. Circ Res 2001: 89(1):E8-14.
15 Qu J, Kryukova Y, Potapova IA, Doronin SV, Larsen M, Krishnamurthy G et al. MiRP1 modulates HCN2 channel expression and gating in cardiac myocytes. J Biol Chem 2004: 279(42):43497-43502.
16 Vinogradova TM, Lyashkov AE, Zhu W, Ruknudin AM, Sirenko S, Yang D et al. High basal protein kinase A-dependent phosphorylation drives rhythmic internal Ca2+ store oscillations and spontaneous beating of cardiac pacemaker cells. Circ Res 2006: 98(4):505-514.
17 Mattick P, Parrington J, Odia E, Simpson A, Collins T, Terrar D. Ca2+-stimulated adenylyl cyclase isoform AC1 is preferentially expressed in guinea-pig sino-atrial node cells and modulates the I(f) pacemaker current. J Physiol 2007: 582(Pt 3):1195-1203.
18 Rose RA, Kabir MG, Backx PH. Altered Heart Rate and Sinoatrial Node Function in Mice Lacking the cAMP Regulator Phosphoinositide 3-Kinase-{gamma}. Circ Res 2007.
19 Kerfant BG, Rose RA, Sun H, Backx PH. Phosphoinositide 3-kinase gamma regulates cardiac contractility by locally controlling cyclic adenosine monophosphate levels. Trends Cardiovasc Med 2006: 16(7):250-256.
20 Mongillo M, McSorley T, Evellin S, Sood A, Lissandron V, Terrin A et al. Fluorescence resonance energy transfer-based analysis of cAMP dynamics in live neonatal rat cardiac myocytes reveals distinct functions of compartmentalized phosphodiesterases. Circ Res 2004: 95(1):67-75.
21 Joyner RW, van Capelle FJ. Propagation through electrically coupled cells. How a small SA node drives a large atrium. Biophys J 1986: 50(6):1157-1164.
22 Joyner RW, Wilders R, Wagner MB. Propagation of pacemaker activity. Med Biol Eng Comput 2006.
23 Rohr S, Kucera JP, Fast VG, Kleber AG. Paradoxical improvement of impulse conduction in cardiac tissue by partial cellular uncoupling. Science 1997: 275(5301):841-844.
24 Seppen J, Rijnberg M, Cooreman MP, Oude Elferink RPJ. Lentiviral vectors for efficient transduction of isolated primary quiescent hepatocytes. J Hepatol 2002: 36(4):459-465.
25 Seppen J, van der Rijt R, Looije N, van Til NP, Lamers WH, Oude Elferink RPJ. Long-term correction of bilirubin UDPglucuronyltransferase deficiency in rats by in utero lentiviral gene transfer. Mol Ther 2003: 8(4):593-599.
26 Rohr S, Schöllly DM, Kléber AG. Patterned growth of neonatal rat heart cells in culture. Morphological and electrophysiological characterization. Circ Res 1991: 68(1):114-130.
27 Ludwig A, Zong X, Jeglitsch M, Hofmann F, Biel M. A family of hyperpolarization-activated mammalian cation channels. Nature 1998: 393(6685):587-591.
28 Er F, Larbig R, Ludwig A, Biel M, Hofmann F, Beuckelmann DJ et al. Dominant-negative suppression of HCN channels markedly reduces the native pacemaker current I(f) and undermines spontaneous beating of neonatal cardiomyocytes. Circulation 2003: 107(3):485-489.
29 van Ginneken AC, Giles W. Voltage clamp measurements of the hyperpolarization-activated inward current I(f) in single cells from rabbit sino-atrial node. J Physiol 1991: 434:57-83.
30 Potse M, Linnenbank AC, Grimbergen CA. Software design for analysis of multichannel intracardial and body surface electrocardiograms. Comput Methods Programs Biomed 2002: 69(3):225-236.
31 Hoogaars WM, Engel A, Brons JF, Verkerk AO, de Lange FJ, Wong LY et al. TBX3 controls the sinoatrial node gene program and imposes pacemaker function on the atria. Genes Dev 2007: 21(9):1098-1112.
32 Brummelkamp TR, Kortlever RM, Lingbeek M, Trettel F, MacDonald ME, van LM et al. TBX-3, the gene mutated in Ulnar-Mammary Syndrome, is a negative regulator of p19ARF and inhibits senescence. J Biol Chem 2002: 277(8):6567-6572.
33 Sohal DS, Nghiem M, Crackower MA, Witt SA, Kimball TR, Tymitz KM et al. Temporally regulated and tissue-specific gene manipulations in the adult and embryonic heart using a tamoxifen-inducible Cre protein. Circ Res 2001: 89(1):20-25.
34 Novak A, Guo C, Yang W, Nagy A, Lobe CG. Z/EG, a double reporter mouse line that expresses enhanced green fluorescent protein upon Cre-mediated excision. Genesis 2000: 28(3-4):147-155.
35 Karlen Y, McNair A, Perseguers S, Mazza C, Mermod N. Statistical significance of quantitative PCR. BMC Bioinformatics 2007: 8:131.

Bucchi A, Plotnikov AN, Shlapakova I, et al. Wild-type and mutant HCN channels in a tandem biological-electronic cardiac pacemaker. Circulation 2006: 114:992-999.

Cai J, Yi FF, Li YH, et al. Adenoviral gene transfer of HCN4 creates a genetic pacemaker in pigs with complete atrioventricular block. Life Sci 2007: 80:1746-1753.

Plotnikov AN, Bucchi A, Shlapakova I, Danilo P Jr, Brink PR, Robinson RB, Cohen IS, Rosen MR. HCN212-channel biological pacemakers manifesting ventricular tachyarrhythmias are responsive to treatment with I(f) blockade. Heart Rhytm 2008: 5: 282-8

Sambrook J, Russell DW, Molecular cloning, a laboratory manual, third edition, 2001 Cold Spring Harbour Laboratory Press, Cold Spring Harbour, New York

## Claims

1. A method for providing a cell with a spontaneous electrical activity and/or increasing the depolarization rate of a cell having a spontaneous electrical activity, the method comprising:
- providing a cell with a compound capable of providing and/or increasing a pacemaker current I_{f}, and
- diminishing electrical coupling between said cell and surrounding cells and/or reducing the inward rectifier current I_{K1} of said cell.

2. A method according to claim 1, wherein said cell is provided with a hyperpolarization-activated cyclic nucleotide-gated (HCN) channel or a functional equivalent thereof.

3. A method according to claim 1 or 2, comprising enhancing the basal cAMP level within said cell.

4. A method according to claim 3, wherein said basal cAMP level is enhanced by increasing the amount and/or activity of a cAMP producing enzyme within said cell.

5. A method according to claim 4, wherein said enzyme comprises an adenylate cyclase.

6. A method according to claim 4 or 5, wherein said enzyme comprises adenylate cyclase-1 and/or adenylate cyclase-8.

7. A method according to any one of claims 3-6, wherein said basal cAMP level is enhanced by reducing the amount and/or activity of an enzyme involved with cAMP breakdown.

8. A method according to claim 7, wherein said enzyme comprises a phosphodiesterase.

9. A method according to any one of claims 1-8, wherein said cell is provided with:
- an siRNA and/or an antisense nucleotide sequence against a phosphodiesterase; and/or
- a nucleic acid sequence or a functional equivalent thereof encoding a phosphodiesterase with a diminished function as compared to wild type phosphodiesterase.

10. A method according to claim 9, wherein said nucleic acid sequence or functional equivalent thereof encodes a phosphodiesterase with a dominant diminished function as compared to wild type phosphodiesterase

11. A method according to any one of claims 1-10, wherein the electrical coupling between said cell and surrounding cells is diminished by providing a barrier between said cell and surrounding cells.

12. A method according to claim 11, wherein said barrier comprises a cell with a reduced conductor capacity as compared to the same kind of cell in a natural situation.

13. A method according to claim 11 or 12, wherein said barrier comprises fibrotic cells.

14. A method according to any one of claims 11-13, wherein said barrier comprises cells which have been heated, preferably cells which have been heated with a device having a temperature of at least 50°C, preferably between 50 and 65°C, more preferably between 55 and 60°C.

15. A method according to any one of claims 11-14, wherein said barrier comprises cells which have been cooled, preferably cells which have been cooled with a device having a temperature of at most -80°C.

16. A method according to any one of claims 1-15, wherein the electrical coupling between said cell and surrounding cells is diminished by reducing the amount and/or activity of gap junction proteins connecting said cell and surrounding cells.

17. A method according to any one of claims 1-16, wherein the electrical coupling between said cell and surrounding cells is diminished by providing said cell with a gap junction protein with a diminished conductor capacity as compared to connexin 43 or connexin 40.

18. A method according to any one of claims 1-17, wherein the electrical coupling between said cell and surrounding cells is diminished by reducing the amount and/or activity of connexin 43 and/or connexin 40 of said cell.

19. A method according to any one of claims 1-18, wherein said cell is provided with:
- an siRNA and/or an antisense nucleotide sequence against connexin 43; and/or
- an siRNA and/or an antisense nucleotide sequence against connexin 40; and/or
- a nucleic acid sequence or a functional equivalent thereof encoding a connexin with a lower conductor capacity than the conductor capacity of connexin 43; and/or
- a nucleic acid sequence or a functional equivalent thereof encoding a connexin with a lower conductor capacity than the conductor capacity of connexin 40.

20. A method according to claim 19, wherein said connexin with a lower conductor capacity as compared to the conductor capacity of connexin 43 or connexin 40 comprises connexin 30.2, connexin 45, connexin43Δ or a functional equivalent thereof.

21. A method according to any one of claims 1-20, wherein said cell is provided with a transcription factor capable of reducing connexin 43 expression and/or connexin 40 expression.

22. A method according to claim 21, wherein said transcription factor comprises TBX3.

23. A method according to any one of claims 1-22, further comprising providing said cell with a nucleic acid sequence or a functional equivalent thereof encoding a beta-subunit for a voltage gated potassium channel.

24. A method according to any one of claims 1-23, wherein the inward rectifier current I_{K1} is reduced by providing said cell with
- an siRNA and/or an antisense nucleotide sequence against an inwardly-rectifying channel; and/or
- a nucleic acid sequence or a functional equivalent thereof encoding an inwardly-rectifying channel with a diminished function as compared to the same kind of inwardly-reetifying channel in a wild type form.

25. A method according to claim 24, wherein said inwardly-rectifying channel comprises a Kir2.1 channel.

26. A method according to any one of claims 1-25, wherein said cell is present in, or brought into, atrial or ventricular myocardium.

27. A vector or an isolated cell comprising:
- a nucleic acid sequence or a functional equivalent thereof encoding a hyperpolarization-activated cyclic nucleotide-gated (HCN) channel, and
- one or more nucleic acid sequences selected from the group consisting of:
an siRNA and/or antisense nucleotide sequence against a phosphodiesterase, an siRNA and/or antisense nucleotide sequence against connexin 43, an siRNA and/or antisense nucleotide sequence against connexin 40, an siRNA and/or antisense nucleotide sequence against an inwardly-rectifying channel, and a nucleic acid sequence or a functional equivalent thereof encoding a compound selected from the group consisting of:
a cAMP producing enzyme, an adenylate cyclase, adenylate cyclase-1, adenylate cyclase-8, a compound capable of increasing the amount and/or activity of a cAMP producing enzyme, a compound capable of reducing the amount and/or activity of an enzyme involved with cAMP breakdown, a phosphodiesterase with a diminished function as compared to wild type phosphodiesterase, a compound capable of reducing the amount and/or activity of gap junction proteins connecting said cell and surrounding cells, a gap junction protein with a diminished conductor capacity as compared to connexin 43, a gap junction protein with a diminished conductor capacity as compared to connexin 40, a compound capable of reducing the amount and/or activity of connexin 43 of said cell, a compound capable of reducing the amount and/or activity of connexin 40 of said cell, a connexin with a lower conductor capacity than the conductor capacity of connexin 43, a connexin with a lower conductor capacity than the conductor capacity of connexin 40, connexin 30.2 or a functional equivalent thereof, connexin 45 or a functional equivalent thereof, connexin 43 Δ or a functional equivalent thereof, a transcription factor capable of reducing connexin 43 expression, a transcription factor capable of reducing connexin 40 expression, TBX3 or a functional equivalent thereof, an inwardly-rectifying potassium channel with a diminished function as compared to the same kind of inwardly-rectifying potassium channel in a wild type form, and a Kir2.1 channel or a functional equivalent thereof.

28. A method or a cell according to any one of claims 1-27, wherein said cell comprises a myocardial cell.

29. A method or a cell according to any one of claims 1-28, wherein said cell comprises a cardiac stem cell or cardiac progenitor cell.

30. A method for treating a subject suffering from, or at risk of suffering from, a disorder associated with impaired function of a cell with a spontaneous electrical activity, the method comprising:
- providing a cell of said subject with spontaneous electrical activity with a method according to any one of claims 1-26, 28 or 29, and/or
- increasing the depolarization rate of a cell of said subject with a method according to any one of claims 1-26, 28 or 29, and/or
- administering to said subject a therapeutic amount of a vector and/or a cell according to claim 27.

31. A method for treating a subject suffering from, or at risk of suffering from, a cardiovascular disorder, the method comprising:
- providing a myocardial cell of said subject with spontaneous electrical activity with a method according to any one of claims 1-26, 28 or 29, and/or
- increasing the depolarization rate of a myocardial cell of said subject with a method according to any one of claims 1-26, 28 or 29, and/or
- administering to said subject a therapeutic amount of a vector and/or a cell according to claim 27.

32. A method according to claim 31, wherein said vector and/or cell is administered to the atrium or the ventricle of the heart of said subject.

33. A method according to claim 31 or 32, wherein said cardiovascular disorder comprises a cardiac conduction disorder, preferably sick sinus syndrome and/or AV nodal block.

34. A device for increasing the depolarization rate of a cell or a group of cells having spontaneous electrical activity, and/or for providing a cell or a group of cells with spontaneous electrical activity, said device comprising:
- means for providing a cell with a compound capable of providing and/or increasing a pacemaker current I_{f}, and
- means for diminishing electrical coupling between said cell and surrounding cells.

35. A device according to claim 34, wherein said device comprises a catheter.

36. A device according to claim 34 or 35, wherein said means for diminishing electrical coupling between said cell and surrounding cells comprises a heating element, preferably an element for radiofrequency ablation, or a cooling element, preferably an element for cryo ablation.

37. A device according to any one of claims 34-36, wherein said means for providing a cell with a compound capable of providing and/or increasing a pacemaker current I_{f} comprises an element for injection of a nucleic acid sequence.

38. A device according to any one of claims 34-37, which has a shape that allows for diminishing, preferably blocking, electrical connections between said cell or group of cells and the surrounding tissues in at least one direction.

39. A combination of:
- a compound capable of providing and/or increasing a pacemaker current I_{f}, and
- a compound capable of diminishing electrical coupling between said cell and surrounding cells and/or a compound capable of reducing the inward rectifier current I_{K1} of said cell for use as a medicament.

40. Use of:
- a compound capable of providing and/or increasing a pacemaker current I_{f}, and
- a compound capable of diminishing electrical coupling between said cell and surrounding cells and/or a compound capable of reducing the inward rectifier current I_{K1} of said cell for the preparation of a medicament for preventing or counteracting a disorder associated with impaired function of a cell with a spontaneous electrical activity, preferably a cardiovascular disorder.

41. A combination or use according to claim 39 or 40, wherein said compound capable of diminishing electrical coupling between said cell and surrounding cells comprises a device according to any one of claims 34-38.

42. A combination or use according to any one of claims 39-41, wherein said compound capable of diminishing electrical coupling between said cell and surrounding cells comprises an siRNA and/or antisense nucleotide sequence against connexin 43 and/or an siRNA and/or antisense nucleotide sequence against connexin 40 and/or a nucleic acid sequence encoding a compound selected from the group consisting of:
a compound capable of reducing the amount and/or activity of gap junction proteins connecting said cell and surrounding cells, a gap junction protein with a diminished conductor capacity as compared to connexin 43, a gap junction protein with a diminished conductor capacity as compared to connexin 40, a compound capable of reducing the amount and/or activity of connexin 43 of said cell, a compound capable of reducing the amount and/or activity of connexin 40 of said cell, a connexin with a lower conductor capacity than the conductor capacity of connexin 43, a connexin with a lower conductor capacity than the conductor capacity of connexin 40, connexin 30.2 or a functional equivalent thereof, connexin 45 or a functional equivalent thereof, connexin 43Δ or a functional equivalent thereof, a transcription factor capable of reducing connexin 43 expression, a transcription factor capable of reducing connexin 40 expression and TBX3 or a functional equivalent thereof.

43. A combination or use according to any one of claims 39-42, wherein said compound capable of providing and/or increasing a pacemaker current I_{f} comprises an siRNA and/or antisense nucleotide sequence against a phosphodiesterase and/or a nucleic acid sequence encoding a compound selected from the group consisting of: a cAMP producing enzyme, an adenylate cyclase, adenylate cyclase-1, adenylate cyclase-8, a compound capable of increasing the amount and/or activity of a cAMP producing enzyme, a compound capable of reducing the amount and/or activity of an enzyme involved with cAMP breakdown, and a phosphodiesterase with a diminished function as compared to wild type phosphodiesterase.

44. A combination or use according to any one of claims 39-43, wherein said compound capable of reducing the inward rectifier current I_{K1} of said cell comprises an siRNA and/or antisense nucleotide sequence against an inwardly-rectifying potassium channel and/or a nucleic acid sequence encoding a compound selected from the group consisting of: an inwardly-rectifying potassium channel with a diminished function as compared to the same kind of inwardly-rectifying potassium channel in a wild type form, and a Kir2.1 channel or a functional equivalent thereof.

45. A pharmaceutical composition, comprising a vector and/or a cell according to claim 27, and a pharmaceutically acceptable carrier, diluent or excipient.
